(19)

**Europäisches Patentamt**
**European Patent Office**
**Office européen des brevets**

(11) **EP 3 937 181 A1**

(12) **EUROPEAN PATENT APPLICATION**

(43) Date of publication:
**12.01.2022 Bulletin 2022/02**

(51) Int Cl.:
***G16H 20/40*** *(2018.01)*

(21) Application number: **21183847.9**

(22) Date of filing: **06.07.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.07.2020 US 202063048830 P**
**29.06.2021 US 202117361600**

(71) Applicant: **Biosense Webster (Israel) Ltd**
**Yokneam, 2066717 (IL)**

(72) Inventors:
- **RAVUNA, Eliyahu**
  **Yokneam (IL)**
- **AMIT, Matitiyahu**
  **Yokneam (IL)**
- **YARNITSKI, Jonathan**
  **Yokneam (IL)**
- **ITAH, Refael**
  **Yokneam (IL)**

- **TSOREF, Liat**
  **Yokneam (IL)**
- **DORON, Itai**
  **Yokneam (IL)**
- **SHALGI, Avi**
  **Yokneam (IL)**
- **ZIV-ARI, Morris**
  **Yokneam (IL)**
- **BOTZER, Lior**
  **Yokneam (IL)**
- **GOLDBERG, Stanislav**
  **Yokneam (IL)**
- **NAKAR, Elad**
  **Yokneam (IL)**
- **COHEN, Assaf**
  **Yokneam (IL)**
- **LEV, Iddo**
  **Yokneam (IL)**

(74) Representative: **Carpmaels & Ransford LLP**
**One Southampton Row**
**London WC1B 5HA (GB)**

(54) **OPTIMIZED ABLATION FOR PERSISTENT ATRIAL FIBRILLATION**

(57)     A method and apparatus of aiding a physician in locating an area to perform an ablation on patients with atrial fibrillation (AFIB) includes receiving data at a machine, from at least one device, the data including information relating to a desired location for performing an ablation, generating, by the machine, an optimal location for performing the ablation based upon the data and inputs, and providing an optimal set of ablation parameters for performing the ablation at the location output by the model, or at a location specified by the physician.

**Description**

CROSS REFERENCE TO RELATED APPLICATIONS

This application claims the benefit of U.S. Provisional Application Serial No. 63/048,830, filed July 7, 2020, which is incorporated by reference as if fully set forth.

FIELD OF INVENTION

The present invention is related to artificial intelligence and machine learning associated with optimizing ablation for persistent atrial fibrillation (AFIB).

BACKGROUND

Atrial arrhythmias are significant contributors for cardiac co-morbidity, especially for stroke, heart failure and recurrent hospitalizations. For some arrhythmias (e.g., typical atrial flutter) the workflow of treatment (ablation) is established in EP community. For other arrhythmias (e.g., persistent atrial fibrillation) the workflow (aside from an anatomy based pulmonary vain isolation - PVI) the treatment (ablation) is not necessarily established due to the complexity of this type of arrhythmia. There are various features that point toward guiding the physicians to where to perform ablation on patients with atrial fibrillation (AFIB), but currently no feature alone can reliably predict the optimal location of the ablation.

SUMMARY

A method and apparatus of aiding a physician in locating an area to perform an ablation on patients with atrial fibrillation (AFIB) includes receiving data at a machine, from at least one device, the data including information relating to a desired location for performing an ablation, generating, by the machine, an optimal location for performing the ablation based upon the data and inputs, and providing an optimal set of ablation parameters for performing the ablation at the location output by the model, or at a location specified by the physician.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 is a block diagram of an example system for remotely monitoring and communicating patient biometrics;
FIG. 2 is a system diagram of an example of a computing environment in communication with network;
FIG. 3 is a block diagram of an example device in which one or more features of the disclosure can be implemented;
FIG. 4 illustrates a graphical depiction of an artificial intelligence system incorporating the example device of FIG. 3;
FIG. 5 illustrates a method performed in the artificial intelligence system of FIG. 4;
FIG. 6 illustrates an example of the probabilities of a naive Bayes calculation;
FIG. 7 illustrates an exemplary decision tree;
FIG. 8 illustrates an exemplary random forest classifier;
FIG. 9 illustrates an exemplary logistic regression;
FIG. 10 illustrates an exemplary support vector machine;
FIG. 11 illustrated an exemplary linear regression model;
FIG. 12 illustrates an exemplary K-means clustering;
FIG. 13 illustrates an exemplary ensemble learning algorithm;
FIG. 14 illustrates an exemplary neural network;
FIG. 15 illustrates a hardware based neural network;
FIG. 16 is a diagram of an exemplary system in which one or more features of the disclosure subject matter can be implemented; and
FIG. 17 is a diagram of a cardiac map of an exemplary retrospective study with locations where a physician performed ablations;
FIG. 18 is a schematic diagram of an exemplary workflow for performing an ablation;
FIG. 19 is an example diagram of an exemplary schematic of aiding a physician in locating an area to perform an ablation on patients with atrial fibrillation (AFIB) using machine learning;
FIG. 20 is an example diagram of an exemplary method of aiding a physician in locating an area to perform an ablation on patients with AFIB using machine learning;

FIG. 21 illustrates the data preparation and training of the present system; and

FIGs. 22 and 23 illustrate an example combined architecture for the present system.

DETAILED DESCRIPTION

Although the details of the application will be described herein, briefly machine learning (ML) is utilized to aid a physician in locating an area to perform an ablation on patients with atrial fibrillation (AFIB).

A method and apparatus of aiding a physician in locating an area to perform an ablation on patients with AFIB includes receiving data at a machine, from at least one device, the data including information relating to a desired location for performing an ablation, generating, by the machine, an optimal location for performing the ablation based upon the data and inputs, and providing an optimal set of ablation parameters for performing the ablation at the location output by the model, or at a location specified by the physician.

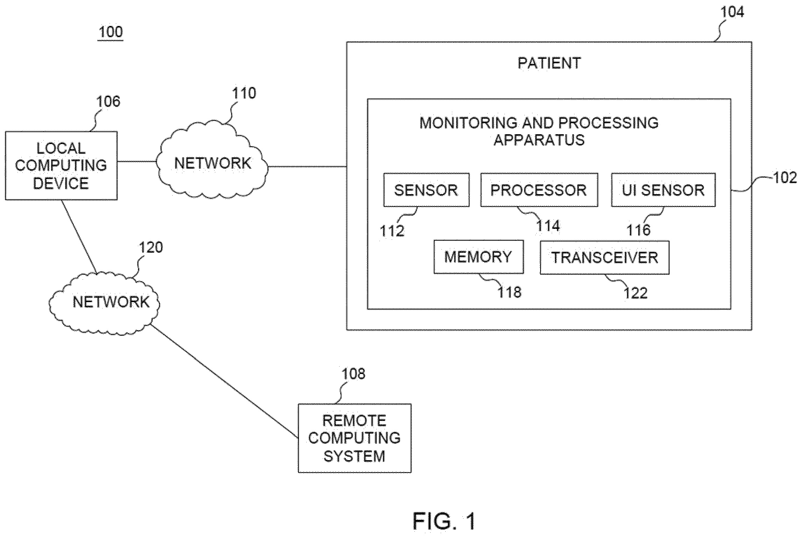

FIG. 1 is a block diagram of an example system 100 for remotely monitoring and communicating patient biometrics (i.e., patient data). In the example illustrated in FIG. 1, the system 100 includes a patient biometric monitoring and processing apparatus 102 associated with a patient 104, a local computing device 106, a remote computing system 108, a first network 110 and a second network 120.

According to an embodiment, a monitoring and processing apparatus 102 may be an apparatus that is internal to the patient's body (e.g., subcutaneously implantable). The monitoring and processing apparatus 102 may be inserted into a patient via any applicable manner including orally injecting, surgical insertion via a vein or artery, an endoscopic procedure, or a laparoscopic procedure.

According to an embodiment, a monitoring and processing apparatus 102 may be an apparatus that is external to the patient. For example, as described in more detail below, the monitoring and processing apparatus 102 may include an attachable patch (e.g., that attaches to a patient's skin). The monitoring and processing apparatus 102 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 102 may include both components that are internal to the patient and components that are external to the patient.

A single monitoring and processing apparatus 102 is shown in FIG. 1. Example systems may, however, may include a plurality of patient biometric monitoring and processing apparatuses. A patient biometric monitoring and processing apparatus may be in communication with one or more other patient biometric monitoring and processing apparatuses. Additionally, or alternatively, a patient biometric monitoring and processing apparatus may be in communication with the network 110.

One or more monitoring and processing apparatuses 102 may acquire patient biometric data (e.g., electrical signals, blood pressure, temperature, blood glucose level or other biometric data) and receive at least a portion of the patient biometric data representing the acquired patient biometrics and additional formation associated with acquired patient biometrics 'from one or more other monitoring and processing apparatuses 102. The additional information may be, for example, diagnosis information and/or additional information obtained from an additional device such as a wearable device. Each monitoring and processing apparatus 102 may process data, including its own acquired patient biometrics as well as data received from one or more other monitoring and processing apparatuses 102.

In FIG. 1, network 110 is an example of a short-range network (e.g., local area network (LAN), or personal area network (PAN)). Information may be sent, via short-range network 110, between monitoring a processing apparatus 102 and local computing device 106 using any one of various short-range wireless communication protocols, such as Bluetooth, Wi-Fi, Zigbee, Z-Wave, near field communications (NFC), ultraband, Zigbee, or infrared (IR).

Network 120 may be a wired network, a wireless network or include one or more wired and wireless networks. For example, a network 120 may be a long-range network (e.g., wide area network (WAN), the internet, or a cellular network,). Information may be sent, via network 120 using any one of various long-range wireless communication protocols (e.g., TCP/IP, HTTP, 3G, 4G/LTE, or 5G/New Radio).

The patient monitoring and processing apparatus 102 may include a patient biometric sensor 112, a processor 114, a user input (UI) sensor 116, a memory 118, and a transmitter-receiver (i.e., transceiver) 122. The patient monitoring and processing apparatus 102 may continually or periodically monitor, store, process and communicate, via network 110, any number of various patient biometrics. Examples of patient biometrics include electrical signals (e.g., ECG signals and brain biometrics), blood pressure data, blood glucose data and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

Patient biometric sensor 112 may include, for example, one or more sensors configured to sense a type of biometric patient biometrics. For example, patient biometric sensor 112 may include an electrode configured to acquire electrical signals (e.g., heart signals, brain signals or other bioelectrical signals), a temperature sensor, a blood pressure sensor, a blood glucose sensor, a blood oxygen sensor, a pH sensor, an accelerometer and a microphone.

As described in more detail below, patient biometric monitoring and processing apparatus 102 may be an ECG monitor for monitoring ECG signals of a heart. The patient biometric sensor 112 of the ECG monitor may include one or more electrodes for acquiring ECG signals. The ECG signals may be used for treatment of various cardiovascular diseases.

In another example, the patient biometric monitoring and processing apparatus 102 may be a continuous glucose monitor (CGM) for continuously monitoring blood glucose levels of a patient on a continual basis for treatment of various diseases, such as type I and type II diabetes. The CGM may include a subcutaneously disposed electrode, which may monitor blood glucose levels from interstitial fluid of the patient. The CGM may be, for example, a component of a closed-loop system in which the blood glucose data is sent to an insulin pump for calculated delivery of insulin without user intervention.

Transceiver 122 may include a separate transmitter and receiver. Alternatively, transceiver 122 may include a transmitter and receiver integrated into a single device.

Processor 114 may be configured to store patient data, such as patient biometric data in memory 118 acquired by patient biometric sensor 112, and communicate the patient data, across network 110, via a transmitter of transceiver 122. Data from one or more other monitoring and processing apparatus 102 may also be received by a receiver of transceiver 122, as described in more detail below.

According to an embodiment, the monitoring and processing apparatus 102 includes UI sensor 116 which may be, for example, a piezoelectric sensor or a capacitive sensor configured to receive a user input, such as a tapping or touching. For example, UI sensor 116 may be controlled to implement a capacitive coupling, in response to tapping or touching a surface of the monitoring and processing apparatus 102 by the patient 104. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface such that the tapping or touching of the surface activates the monitoring device.

As described in more detail below, the processor 114 may be configured to respond selectively to different tapping patterns of the capacitive sensor (e.g., a single tap or a double tap), which may be the UI sensor 116, such that different tasks of the patch (e.g., acquisition, storing, or transmission of data) may be activated based on the detected pattern. In some embodiments, audible feedback may be given to the user from processing apparatus 102 when a gesture is detected.

The local computing device 106 of system 100 is in communication with the patient biometric monitoring and processing apparatus 102 and may be configured to act as a gateway to the remote computing system 108 through the second network 120. The local computing device 106 may be, for example, a, smart phone, smartwatch, tablet or other portable smart device configured to communicate with other devices via network 120. Alternatively, the local computing device 106 may be a stationary or standalone device, such as a stationary base station including, for example, modem and/or router capability, a desktop or laptop computer using an executable program to communicate information between the processing apparatus 102 and the remote computing system 108 via the PC's radio module, or a USB dongle. Patient biometrics may be communicated between the local computing device 106 and the patient biometric monitoring and processing apparatus 102 using a short-range wireless technology standard (e.g., Bluetooth, Wi-Fi, ZigBee, Z-wave and other short-range wireless standards) via the short-range wireless network 110, such as a local area network (LAN) (e.g., a personal area network (PAN)). In some embodiments, the local computing device 106 may also be configured to display the acquired patient electrical signals and information associated with the acquired patient electrical signals, as described in more detail below.

In some embodiments, remote computing system 108 may be configured to receive at least one of the monitored patient biometrics and information associated with the monitored patient via network 120, which is a long-range network. For example, if the local computing device 106 is a mobile phone, network 120 may be a wireless cellular network, and information may be communicated between the local computing device 106 and the remote computing system 108 via a wireless technology standard, such as any of the wireless technologies mentioned above. As described in more detail below, the remote computing system 108 may be configured to provide (e.g., visually display and/or aurally provide) the at least one of the patient biometrics and the associated information to a healthcare professional (e.g., a physician).

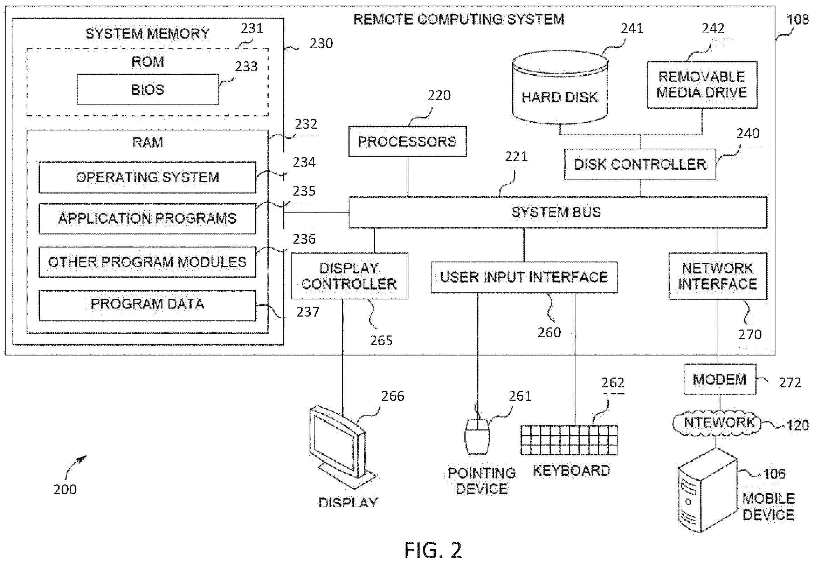

FIG. 2 is a system diagram of an example of a computing environment 200 in communication with network 120. In some instances, the computing environment 200 is incorporated in a public cloud computing platform (such as Amazon Web Services or Microsoft Azure), a hybrid cloud computing platform (such as HP Enterprise OneSphere) or a private cloud computing platform.

As shown in FIG. 2, computing environment 200 includes remote computing system 108 (hereinafter computer system), which is one example of a computing system upon which embodiments described herein may be implemented.

The remote computing system 108 may, via processors 220, which may include one or more processors, perform various functions. The functions may include analyzing monitored patient biometrics and the associated information and, according to physician-determined or algorithm driven thresholds and parameters, providing (e.g., via display 266) alerts, additional information or instructions. As described in more detail below, the remote computing system 108 may be used to provide (e.g., via display 266) healthcare personnel (e.g., a physician) with a dashboard of patient information, such that such information may enable healthcare personnel to identify and prioritize patients having more critical needs than others.

As shown in FIG. 2, the computer system 210 may include a communication mechanism such as a bus 221 or other communication mechanism for communicating information within the computer system 210. The computer system 210 further includes one or more processors 220 coupled with the bus 221 for processing the information. The processors 220 may include one or more CPUs, GPUs, TPUs, or any other processor known in the art.

The computer system 210 also includes a system memory 230 coupled to the bus 221 for storing information and instructions to be executed by processors 220. The system memory 230 may include computer readable storage media in the form of volatile and/or nonvolatile memory, such as read only system memory (ROM) 231 and/or random-access memory (RAM) 232. The system memory RAM 232 may include other dynamic storage device(s) (e.g., dynamic RAM, static RAM, and synchronous DRAM). The system memory ROM 231 may include other static storage device(s) (e.g., programmable ROM, erasable PROM, and electrically erasable PROM). In addition, the system memory 230 may be used for storing temporary variables or other intermediate information during the execution of instructions by the processors 220. A basic input/output system 233 (BIOS) may contain routines to transfer information between elements within computer system 210, such as during start-up, that may be stored in system memory ROM 231. RAM 232 may comprise data and/or program modules that are immediately accessible to and/or presently being operated on by the processors 220. System memory 230 may additionally include, for example, operating system 234, application programs 235, other program modules 236 and program data 237.

The illustrated computer system 210 also includes a disk controller 240 coupled to the bus 221 to control one or more storage devices for storing information and instructions, such as a magnetic hard disk 241 and a removable media drive 242 (e.g., floppy disk drive, compact disc drive, tape drive, and/or solid-state drive). The storage devices may be added to the computer system 210 using an appropriate device interface (e.g., a small computer system interface (SCSI), integrated device electronics (IDE), Universal Serial Bus (USB), or FireWire).

The computer system 210 may also include a display controller 265 coupled to the bus 221 to control a monitor or display 266, such as a cathode ray tube (CRT) or liquid crystal display (LCD), for displaying information to a computer user. The illustrated computer system 210 includes a user input interface 260 and one or more input devices, such as a keyboard 262 and a pointing device 261, for interacting with a computer user and providing information to the processor 220. The pointing device 261, for example, may be a mouse, a trackball, or a pointing stick for communicating direction information and command selections to the processor 220 and for controlling cursor movement on the display 266. The display 266 may provide a touch screen interface that may allow input to supplement or replace the communication of direction information and command selections by the pointing device 261 and/or keyboard 262.

The computer system 210 may perform a portion or each of the functions and methods described herein in response to the processors 220 executing one or more sequences of one or more instructions contained in a memory, such as the system memory 230. Such instructions may be read into the system memory 230 from another computer readable medium, such as a hard disk 241 or a removable media drive 242. The hard disk 241 may contain one or more data stores and data files used by embodiments described herein. Data store contents and data files may be encrypted to improve security. The processors 220 may also be employed in a multiprocessing arrangement to execute the one or more sequences of instructions contained in system memory 230. In alternative embodiments, hard-wired circuitry may be used in place of or in combination with software instructions. Thus, embodiments are not limited to any specific combination of hardware circuitry and software.

As stated above, the computer system 210 may include at least one computer readable medium or memory for holding instructions programmed according to embodiments described herein and for containing data structures, tables, records, or other data described herein. The term computer readable medium as used herein refers to any non-transitory, tangible medium that participates in providing instructions to the processor 220 for execution. A computer readable medium may take many forms including, but not limited to, non-volatile media, volatile media, and transmission media. Non-limiting examples of non-volatile media include optical disks, solid state drives, magnetic disks, and magneto-optical disks, such as hard disk 241 or removable media drive 242. Non-limiting examples of volatile media include dynamic memory, such as system memory 230. Non-limiting examples of transmission media include coaxial cables, copper wire, and fiber optics, including the wires that make up the bus 221. Transmission media may also take the form of acoustic or light waves, such as those generated during radio wave and infrared data communications.

The computing environment 200 may further include the computer system 210 operating in a networked environment using logical connections to local computing device 106 and one or more other devices, such as a personal computer (laptop or desktop), mobile devices (e.g., patient mobile devices), a server, a router, a network PC, a peer device or other common network node, and typically includes many or all of the elements described above relative to computer system 210. When used in a networking environment, computer system 210 may include modem 272 for establishing communications over a network 120, such as the Internet. Modem 272 may be connected to system bus 221 via network interface 270, or via another appropriate mechanism.

Network 120, as shown in FIGs. 1 and 2, may be any network or system generally known in the art, including the Internet, an intranet, a local area network (LAN), a wide area network (WAN), a metropolitan area network (MAN), a direct connection or series of connections, a cellular telephone network, or any other network or medium capable of facilitating communication between computer system 610 and other computers (e.g., local computing device 106).

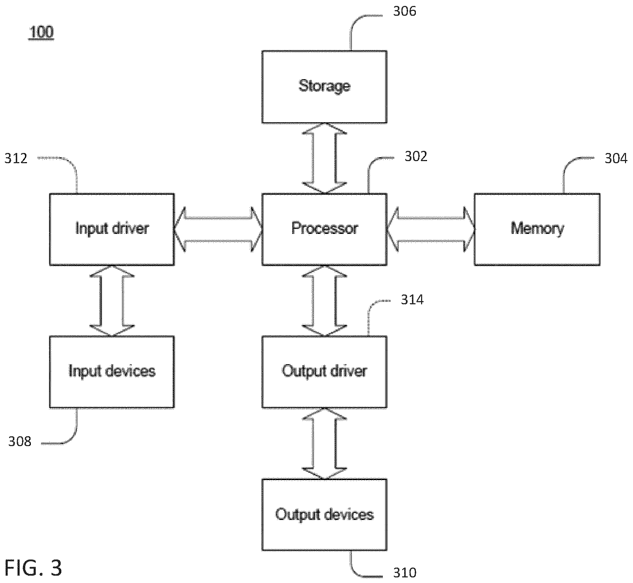

FIG. 3 is a block diagram of an example device 300 in which one or more features of the disclosure can be implemented. The device 300 may be local computing device 106, for example. The device 300 can include, for example, a computer, a gaming device, a handheld device, a set-top box, a television, a mobile phone, or a tablet computer. The device 300 includes a processor 302, a memory 304, a storage device 306, one or more input devices 308, and one or more output devices 310. The device 300 can also optionally include an input driver 312 and an output driver 314. It is understood that the device 300 can include additional components not shown in FIG. 3 including an artificial intelligence accelerator.

In various alternatives, the processor 302 includes a central processing unit (CPU), a graphics processing unit (GPU), a CPU and GPU located on the same die, or one or more processor cores, wherein each processor core can be a CPU or a GPU. In various alternatives, the memory 304 is located on the same die as the processor 302, or is located separately from the processor 302. The memory 304 includes a volatile or non-volatile memory, for example, random access memory (RAM), dynamic RAM, or a cache.

The storage device 306 includes a fixed or removable storage means, for example, a hard disk drive, a solid-state drive, an optical disk, or a flash drive. The input devices 308 include, without limitation, a keyboard, a keypad, a touch screen, a touch pad, a detector, a microphone, an accelerometer, a gyroscope, a biometric scanner, or a network connection (e.g., a wireless local area network card for transmission and/or reception of wireless IEEE 802 signals). The output devices 310 include, without limitation, a display, a speaker, a printer, a haptic feedback device, one or more lights, an antenna, or a network connection (e.g., a wireless local area network card for transmission and/or reception of wireless IEEE 802 signals).

The input driver 312 communicates with the processor 302 and the input devices 308, and permits the processor 302 to receive input from the input devices 308. The output driver 314 communicates with the processor 302 and the output devices 310, and permits the processor 302 to send output to the output devices 310. It is noted that the input driver 312 and the output driver 314 are optional components, and that the device 300 will operate in the same manner if the input driver 312 and the output driver 314 are not present. The output driver 316 includes an accelerated processing device ("APD") 316 which is coupled to a display device 318. The APD accepts compute commands and graphics rendering commands from processor 302, processes those compute and graphics rendering commands, and provides pixel output to display device 318 for display. As described in further detail below, the APD 316 includes one or more parallel processing units to perform computations in accordance with a single-instruction-multiple-data ("SIMD") paradigm. Thus, although various functionality is described herein as being performed by or in conjunction with the APD 316, in various alternatives, the functionality described as being performed by the APD 316 is additionally or alternatively performed by other computing devices having similar capabilities that are not driven by a host processor (e.g., processor 302) and provides graphical output to a display device 318. For example, it is contemplated that any processing system that performs processing tasks in accordance with a SIMD paradigm may perform the functionality described herein. Alternatively, it is contemplated that computing systems that do not perform processing tasks in accordance with a SIMD paradigm performs the functionality described herein.

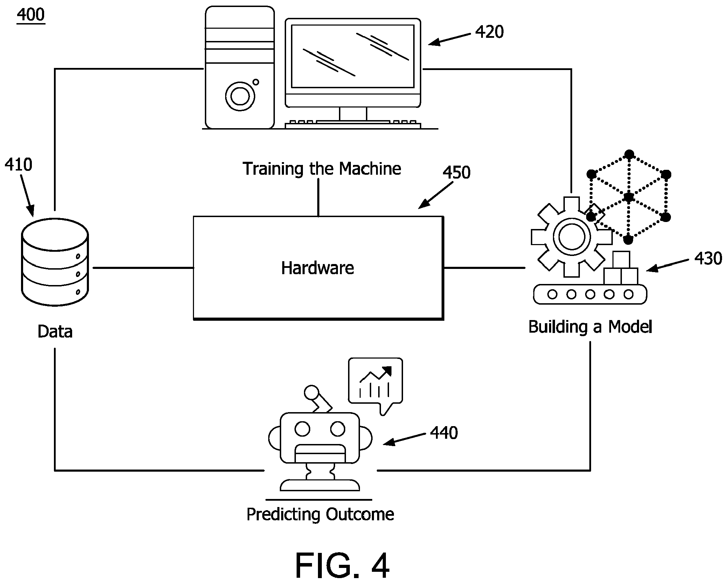

FIG. 4 illustrates a graphical depiction of an artificial intelligence system 200 incorporating the example device of FIG. 3. System 400 includes data 410, a machine 420, a model 430, a plurality of outcomes 440 and underlying hardware 450. System 400 operates by using the data 410 to train the machine 420 while building a model 430 to enable a plurality of outcomes 440 to be predicted. The system 400 may operate with respect to hardware 450. In such a configuration, the data 410 may be related to hardware 450 and may originate with apparatus 102, for example. For example, the data 410 may be on-going data, or output data associated with hardware 450. The machine 420 may operate as the controller or data collection associated with the hardware 450, or be associated therewith. The model 430 may be configured to model the operation of hardware 450 and model the data 410 collected from hardware 450 in order to predict the outcome achieved by hardware 450. Using the outcome 440 that is predicted, hardware 450 may be configured to provide a certain desired outcome 440 from hardware 450.

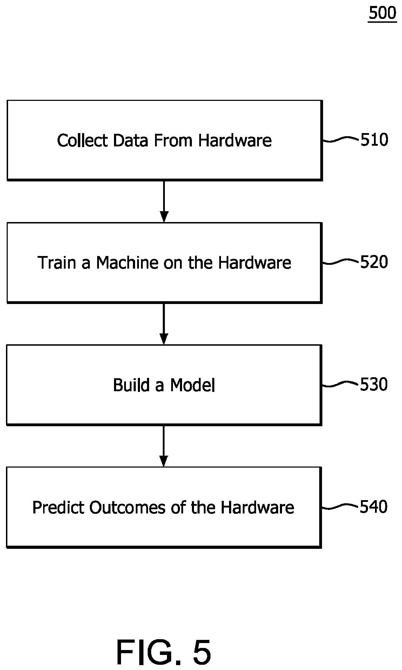

FIG. 5 illustrates a method 500 performed in the artificial intelligence system of FIG. 4. Method 500 includes collecting data from the hardware at step 510. This data may include currently collected, historical or other data from the hardware. For example, this data may include measurements during a surgical procedure and may be associated with the outcome of the procedure. For example, the temperature of a heart may be collected and correlated with the outcome of a heart procedure.

At step 520, method 500 includes training a machine on the hardware. The training may include an analysis and correlation of the data collected in step 510. For example, in the case of the heart, the data of temperature and outcome may be trained to determine if a correlation or link exists between the temperature of the heart during the procedure and the outcome.

At step 530, method 500 includes building a model on the data associated with the hardware. Building a model may include physical hardware or software modeling, algorithmic modeling and the like, as will be described below. This modeling may seek to represent the data that has been collected and trained.

At step 540, method 500 includes predicting the outcomes of the model associated with the hardware. This prediction of the outcome may be based on the trained model. For example, in the case of the heart, if the temperature during the procedure between 97.7 - 100.2 produces a positive result from the procedure, the outcome can be predicted in a given procedure based on the temperature of the heart during the procedure. While this model is rudimentary, it is provided for exemplary purposes and to increase understanding of the present invention.

The present system and method operate to train the machine, build the model and predict outcomes using algorithms. These algorithms may be used to solve the trained model and predict outcomes associated with the hardware. These algorithms may be divided generally into classification, regression and clustering algorithms.

For example, a classification algorithm is used in the situation where the dependent variable, which is the variable being predicted, is divided into classes and predicting a class, the dependent variable, for a given input. Thus, a classification algorithm is used to predict an outcome, from a set number of fixed, predefined outcomes. A classification algorithm may include naive Bayes algorithms, decision trees, random forest classifiers, logistic regressions, support vector machines and k nearest neighbors.

Generally, a naive Bayes algorithm follows the Bayes theorem, and follows a probabilistic approach. As would be understood, other probabilistic-based algorithms may also be used, and generally operate using similar probabilistic principles to those described below for the exemplary naive Bayes algorithm.

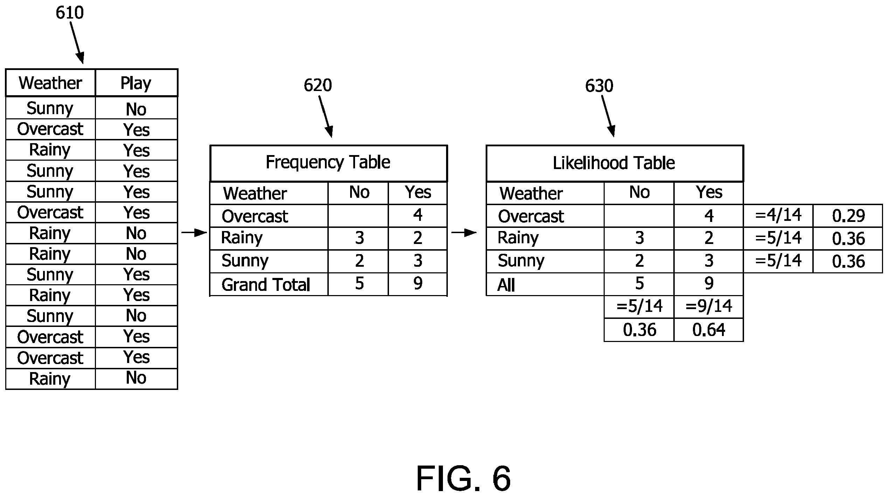

FIG. 6 illustrates an example of the probabilities of a naive Bayes calculation. The probability approach of Bayes theorem essentially means, that instead of jumping straight into the data, the algorithm has a set of prior probabilities for each of the classes for the target. After the data is entered, the naive Bayes algorithm may update the prior probabilities to form a posterior probability. This is given by the formula:

$$posterior = \frac{prior \ x \ likelihood}{evidence}$$

This naive Bayes algorithm, and Bayes algorithms generally, may be useful when needing to predict whether your input belongs to a given list of n classes or not. The probabilistic approach may be used because the probabilities for all the n classes will be quite low.

For example, as illustrated in FIG. 6, a person playing golf, which depends on factors including the weather outside shown in a first data set 610. The first data set 610 illustrates the weather in a first column and an outcome of playing associated with that weather in a second column. In the frequency table 620 the frequencies with which certain events occur are generated. In frequency table 620, the frequency of a person playing or not playing golf in each of the weather conditions is determined. From there, a likelihood table is compiled to generate initial probabilities. For example, the probability of the weather being overcast is 0.29 while the general probability of playing is 0.64.

The posterior probabilities may be generated from the likelihood table 630. These posterior probabilities may be configured to answer questions about weather conditions and whether golf is played in those weather conditions. For example, the probability of it being sunny outside and golf being played may be set forth by the Bayesian formula:

$$P(Yes \mid Sunny) = P(Sunny \mid Yes) * P(Yes) / P(Sunny)$$

According to likelihood table 630:

P (Sunny | Yes) = 3/9 = 0.33,
P(Sunny) = 5/14 = 0.36,
P(Yes)= 9/14 = 0.64.

Therefore, the P(Yes | Sunny) = .33*.64/.36 or approximately 0.60 (60%).

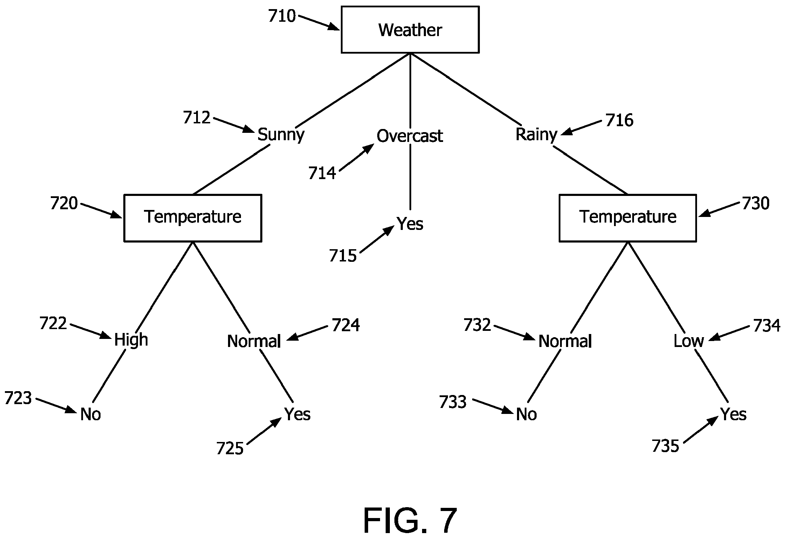

Generally, a decision tree is a flowchart-like tree structure where each external node denotes a test on an attribute and each branch represents the outcome of that test. The leaf nodes contain the actual predicted labels. The decision tree begins from the root of the tree with attribute values being compared until a leaf node is reached. A decision tree can be used as a classifier when handling high dimensional data and when little time has been spent behind data preparation. Decision trees may take the form of a simple decision tree, a linear decision tree, an algebraic decision tree, a deterministic decision tree, a randomized decision tree, a nondeterministic decision tree, and a quantum decision tree. An exemplary decision tree is provided below in FIG. 7.

FIG. 7 illustrates a decision tree, along the same structure as the Bayes example above, in deciding whether to play golf. In the decision tree, the first node 710 examines the weather providing sunny 712, overcast 714, and rain 716 as the choices to progress down the decision tree. If the weather is sunny, the leg of the tree is followed to a second node 720 examining the temperature. The temperature at node 720 may be high 722 or normal 724, in this example. If the temperature at node 720 is high 722, then the predicted outcome of "No" 723 golf occurs. If the temperature at node 720 is normal 724, then the predicted outcome of "Yes" 725 golf occurs.

Further, from the first node 710, an outcome overcast 714, "Yes" 715 golf occurs.

From the first node weather 710, an outcome of rain 716 results in the third node 730 (again) examining temperature. If the temperature at third node 730 is normal 732, then "Yes" 733 golf is played. If the temperature at third node 730 is low 734, then "No" 735 golf is played.

From this decision tree, a golfer plays golf if the weather is overcast 715, in normal temperature sunny weather 725, and in normal temperature rainy weather 733, while the golfer does not play if there are sunny high temperatures 723 or low rainy temperatures 735.

A random forest classifier is a committee of decision trees, where each decision tree has been fed a subset of the attributes of data and predicts on the basis of that subset. The mode of the actual predicted values of the decision trees are considered to provide an ultimate random forest answer. The random forest classifier, generally, alleviates overfitting, which is present in a standalone decision tree, leading to a much more robust and accurate classifier.

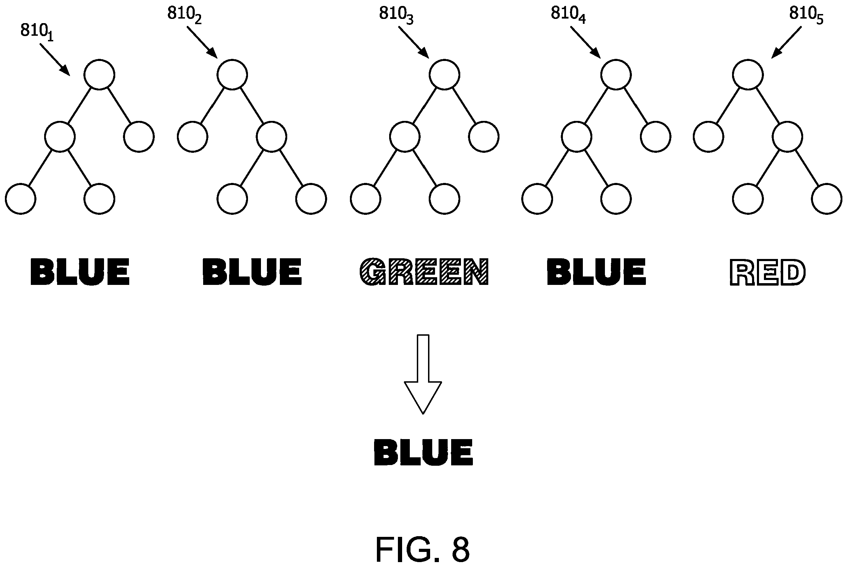

FIG. 8 illustrates an exemplary random forest classifier for classifying the color of a garment. As illustrated in FIG. 8, the random forest classifier includes five decision trees $810_1$, $810_2$, $810_3$, $810_4$, and $810_5$ (collectively or generally referred to as decision trees 810). Each of the trees is designed to classify the color of the garment. A discussion of each of the trees and decisions made is not provided, as each individual tree generally operates as the decision tree of FIG. 7. In the illustration, three ($810_1$, $810_2$, $810_4$) of the five trees determines that the garment is blue, while one determines the garment is green ($810_3$) and the remaining tree determines the garment is red ($810_5$). The random forest takes these actual predicted values of the five trees and calculates the mode of the actual predicted values to provide random forest answer that the garment is blue.

Logistic Regression is another algorithm for binary classification tasks. Logistic regression is based on the logistic function, also called the sigmoid function. This S-shaped curve can take any real-valued number and map it between 0 and 1 asymptotically approaching those limits. The logistic model may be used to model the probability of a certain class or event existing such as pass/fail, win/lose, alive/dead or healthy/sick. This can be extended to model several classes of events such as determining whether an image contains a cat, dog, lion, etc. Each object being detected in the image would be assigned a probability between 0 and 1 with the sum of the probabilities adding to one.

In the logistic model, the log-odds (the logarithm of the odds) for the value labeled "1" is a linear combination of one or more independent variables ("predictors"); the independent variables can each be a binary variable (two classes, coded by an indicator variable) or a continuous variable (any real value). The corresponding probability of the value labeled "1" can vary between 0 (certainly the value "0") and 1 (certainly the value "1"), hence the labeling; the function that converts log-odds to probability is the logistic function, hence the name. The unit of measurement for the log-odds scale is called a logit, from logistic unit, hence the alternative names. Analogous models with a different sigmoid function instead of the logistic function can also be used, such as the probit model; the defining characteristic of the logistic model is that increasing one of the independent variables multiplicatively scales the odds of the given outcome at a constant rate, with each independent variable having its own parameter; for a binary dependent variable this generalizes the odds ratio.

In a binary logistic regression model, the dependent variable has two levels (categorical). Outputs with more than two values are modeled by multinomial logistic regression and, if the multiple categories are ordered, by ordinal logistic regression (for example the proportional odds ordinal logistic model). The logistic regression model itself simply models probability of output in terms of input and does not perform statistical classification (it is not a classifier), though it can be used to make a classifier, for instance by choosing a cutoff value and classifying inputs with probability greater than the cutoff as one class, below the cutoff as the other; this is a common way to make a binary classifier.

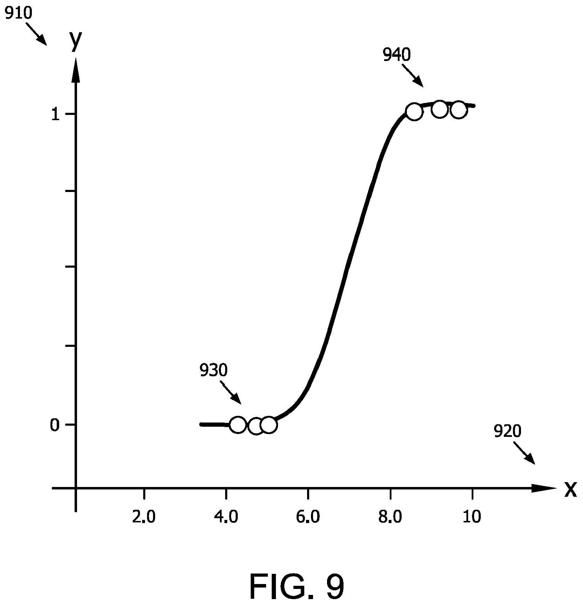

FIG. 9 illustrates an exemplary logistic regression. This exemplary logistic regression enables the prediction of an outcome based on a set of variables. For example, based on a person's grade point average, and outcome of being accepted to a school may be predicted. The past history of grade point averages and the relationship with acceptance enables the prediction to occur. The logistic regression of FIG. 9 enables the analysis of the grade point average variable 920 to predict the outcome 910 defined by 0 to 1. At the low end 930 of the S-shaped curve, the grade point average 920 predicts an outcome 910 of not being accepted. While at the high end 940 of the S-shaped curve, the grade point average 920 predicts an outcome 910 of being accepted. Logistic regression may be used to predict house values, customer lifetime value in the insurance sector, etc.

A support vector machine (SVM) may be used to sort the data with the margins between two classes as far apart as possible. This is called maximum margin separation. The SVM may account for the support vectors while plotting the hyperplane, unlike linear regression which uses the entire dataset for that purpose.

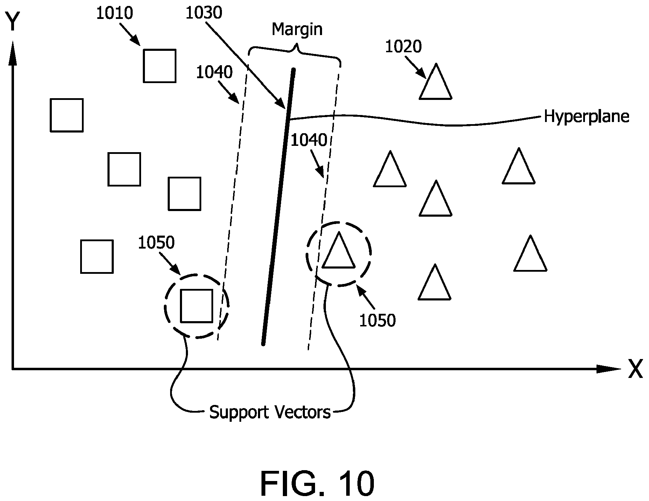

FIG. 10 illustrates an exemplary support vector machine. In the exemplary SVM 1000, data may be classified into two different classes represented as squares 1010 and triangles 1020. SVM 1000 operates by drawing a random hyperplane 1030. This hyperplane 1030 is monitored by comparing the distance (illustrated with lines 1040) between the hyperplane 1030 and the closest data points 1050 from each class. The closest data points 1050 to the hyperplane 1030 are known as support vectors. The hyperplane 1030 is drawn based on these support vectors 1050 and an optimum hyperplane has a maximum distance from each of the support vectors 1050. The distance between the hyperplane 1030 and the support vectors 1050 is known as the margin.

SVM 1000 may be used to classify data by using a hyperplane 1030, such that the distance between the hyperplane 1030 and the support vectors 1050 is maximum. Such an SVM 1000 may be used to predict heart disease, for example.

K Nearest Neighbors (KNN) refers to a set of algorithms that generally do not make assumptions on the underlying data distribution, and perform a reasonably short training phase. Generally, KNN uses many data points separated into several classes to predict the classification of a new sample point. Operationally, KNN specifies an integer N with a new sample. The N entries in the model of the system closest to the new sample are selected. The most common classification of these entries is determined and that classification is assigned to the new sample. KNN generally requires the storage space to increase as the training set increases. This also means that the estimation time increases in proportion to the number of training points.

In regression algorithms, the output is a continuous quantity so regression algorithms may be used in cases where the target variable is a continuous variable. Linear regression is a general example of regression algorithms. Linear regression may be used to gauge genuine qualities (cost of houses, number of calls, all out deals and so forth) in view of the consistent variable(s). A connection between the variables and the outcome is created by fitting the best line (hence linear regression). This best fit line is known as regression line and spoken to by a direct condition $Y= a *X + b$. Linear regression is best used in approaches involving a low number of dimensions

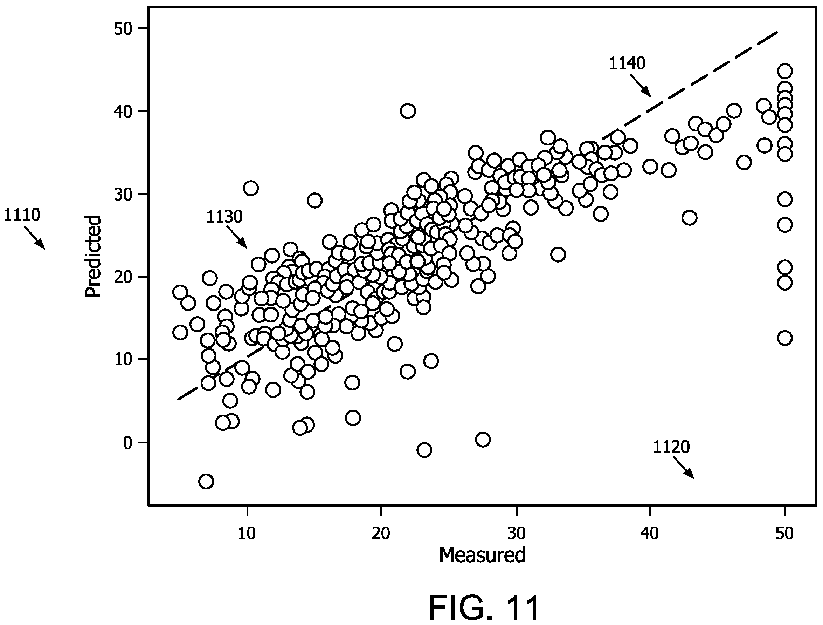

FIG. 11 illustrates an exemplary linear regression model. In this model, a predicted variable 1110 is modeled against a measured variable 1120. A cluster of instances of the predicted variable 1110 and measured variable 1120 are plotted as data points 1130. Data points 1130 are then fit with the best fit line 1140. Then the best fit line 1140 is used in subsequent predicted, given a measured variable 1120, the line 1140 is used to predict the predicted variable 1110 for that instance. Linear regression may be used to model and predict in a financial portfolio, salary forecasting, real estate and in traffic in arriving at estimated time of arrival.

Clustering algorithms may also be used to model and train on a data set. In clustering, the input is assigned into two or more clusters based on feature similarity. Clustering algorithms generally learn the patterns and useful insights from data without any guidance. For example, clustering viewers into similar groups based on their interests, age, geography, etc. may be performed using unsupervised learning algorithms like K-means clustering.

K-means clustering generally is regarded as a simple unsupervised learning approach. In K-means clustering similar data points may be gathered together and bound in the form of a cluster. One method for binding the data points together is by calculating the centroid of the group of data points. In determining effective clusters, in K-means clustering the distance between each point from the centroid of the cluster is evaluated. Depending on the distance between the data point and the centroid, the data is assigned to the closest cluster. The goal of clustering is to determine the intrinsic grouping in a set of unlabeled data. The 'K' in K-means stands for the number of clusters formed. The number of clusters (basically the number of classes in which new instances of data may be classified) may be determined by the user. This determination may be performed using feedback and viewing the size of the clusters during training, for example.

K-means is used majorly in cases where the data set has points which are distinct and well separated, otherwise, if the clusters are not separated the modeling may render the clusters inaccurate. Also, K-means may be avoided in cases where the data set contains a high number of outliers or the data set is non-linear.

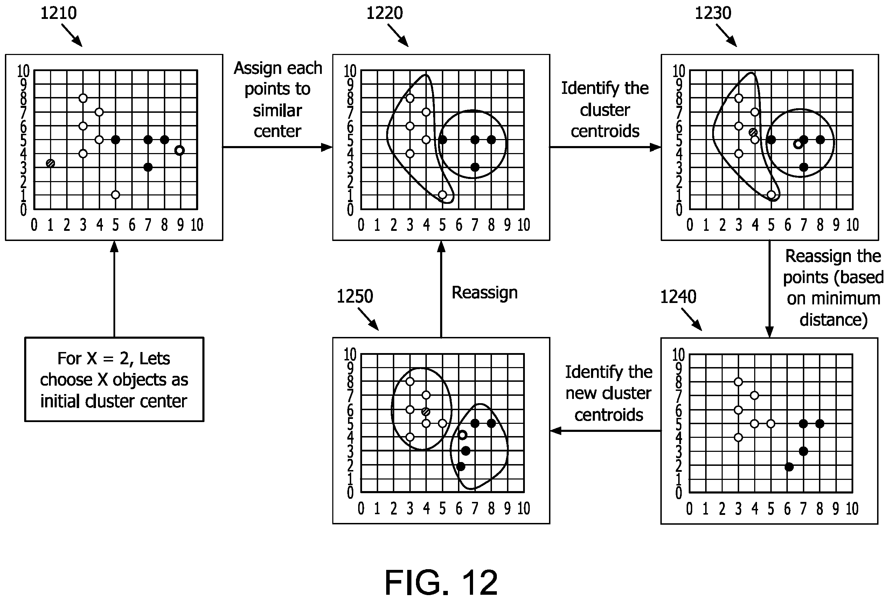

FIG. 12 illustrates a K-means clustering. In K-means clustering, the data points are plotted and the K value is assigned. For example, for K=2 in FIG. 12, the data points are plotted as shown in depiction 1210. The points are then assigned to similar centers at step 1220. The cluster centroids are identified as shown in 1230. Once centroids are identified, the points are reassigned to the cluster to provide the minimum distance between the data point to the respective cluster centroid as illustrated in 1240. Then a new centroid of the cluster may be determined as illustrated in depiction 1250. As the data pints are reassigned to a cluster, new cluster centroids formed, an iteration, or series of iterations, may occur to enable the clusters to be minimized in size and the centroid of the optimal centroid determined. Then as new data points are measured, the new data points may be compared with the centroid and cluster to identify with that cluster.

Ensemble learning algorithms may be used. These algorithms use multiple learning algorithms to obtain better predictive performance than could be obtained from any of the constituent learning algorithms alone. Ensemble learning algorithms perform the task of searching through a hypothesis space to find a suitable hypothesis that will make good predictions with a particular problem. Even if the hypothesis space contains hypotheses that are very well-suited for a particular problem, it may be very difficult to find a good hypothesis. Ensemble algorithms combine multiple hypotheses to form a better hypothesis. The term ensemble is usually reserved for methods that generate multiple hypotheses using the same base learner. The broader term of multiple classifier systems also covers hybridization of hypotheses that are not induced by the same base learner.

Evaluating the prediction of an ensemble typically requires more computation than evaluating the prediction of a single model, so ensembles may be thought of as a way to compensate for poor learning algorithms by performing a lot of extra computation. Fast algorithms such as decision trees are commonly used in ensemble methods, for example, random forests, although slower algorithms can benefit from ensemble techniques as well.

An ensemble is itself a supervised learning algorithm, because it can be trained and then used to make predictions. The trained ensemble, therefore, represents a single hypothesis. This hypothesis, however, is not necessarily contained within the hypothesis space of the models from which it is built. Thus, ensembles can be shown to have more flexibility in the functions they can represent. This flexibility can, in theory, enable them to over-fit the training data more than a single model would, but in practice, some ensemble techniques (especially bagging) tend to reduce problems related to over-fitting of the training data.

Empirically, ensemble algorithms tend to yield better results when there is a significant diversity among the models. Many ensemble methods, therefore, seek to promote diversity among the models they combine. Although non-intuitive, more random algorithms (like random decision trees) can be used to produce a stronger ensemble than very deliberate algorithms (like entropy-reducing decision trees). Using a variety of strong learning algorithms, however, has been shown to be more effective than using techniques that attempt to dumb-down the models in order to promote diversity.

The number of component classifiers of an ensemble has a great impact on the accuracy of prediction. *A priori* determining of ensemble size and the volume and velocity of big data streams make this even more crucial for online ensemble classifiers. A theoretical framework suggests that there are an ideal number of component classifiers for an ensemble such that having more or less than this number of classifiers would deteriorate the accuracy. The theoretical framework shows that using the same number of independent component classifiers as class labels gives the highest accuracy.

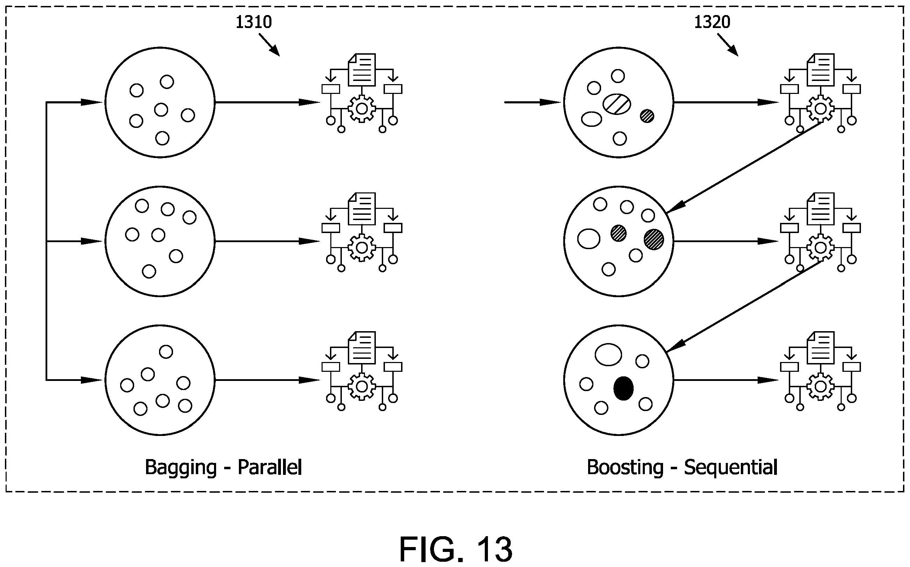

Some common types of ensembles include Bayes optimal classifier, bootstrap aggregating (bagging), boosting, Bayesian model averaging, Bayesian model combination, bucket of models and stacking. FIG. 13 illustrates an exemplary ensemble learning algorithm where bagging is being performed in parallel 1310 and boosting is being performed sequentially 1320.

A neural network is a network or circuit of neurons, or in a modern sense, an artificial neural network, composed of artificial neurons or nodes. The connections of the biological neuron are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

These artificial networks may be used for predictive modeling, adaptive control and applications and can be trained via a dataset. Self-learning resulting from experience can occur within networks, which can derive conclusions from a complex and seemingly unrelated set of information.

For completeness, a biological neural network is composed of a group or groups of chemically connected or functionally associated neurons. A single neuron may be connected to many other neurons and the total number of neurons and connections in a network may be extensive. Connections, called synapses, are usually formed from axons to dendrites, though dendrodendritic synapses and other connections are possible. Apart from the electrical signaling, there are other forms of signaling that arise from neurotransmitter diffusion.

Artificial intelligence, cognitive modeling, and neural networks are information processing paradigms inspired by the way biological neural systems process data. Artificial intelligence and cognitive modeling try to simulate some properties of biological neural networks. In the artificial intelligence field, artificial neural networks have been applied successfully to speech recognition, image analysis and adaptive control, in order to construct software agents (in computer and video games) or autonomous robots.

A neural network (NN), in the case of artificial neurons called artificial neural network (ANN) or simulated neural network (SNN), is an interconnected group of natural or artificial neurons that uses a mathematical or computational model for information processing based on a connectionistic approach to computation. In most cases an ANN is an adaptive system that changes its structure based on external or internal information that flows through the network. In more practical terms neural networks are non-linear statistical data modeling or decision-making tools. They can be used to model complex relationships between inputs and outputs or to find patterns in data.

An artificial neural network involves a network of simple processing elements (artificial neurons) which can exhibit complex global behavior, determined by the connections between the processing elements and element parameters.

One classical type of artificial neural network is the recurrent Hopfield network. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which artificial neural networks are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

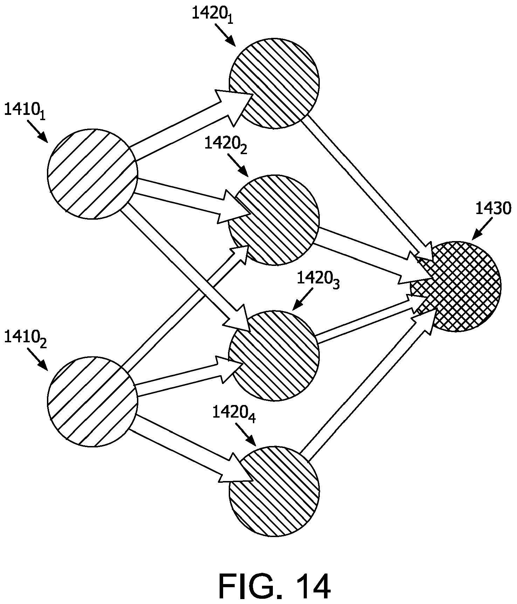

FIG. 14 illustrates an exemplary neural network. In the neural network there is an input layer represented by a plurality of inputs, such as $1410_1$ and $1410_2$. The inputs $1410_1$, $1410_2$ are provided to a hidden layer depicted as including nodes $1420_1$, $1420_2$, $1420_3$, $1420_4$. These nodes $1420_1$, $1420_2$, $1420_3$, $1420_4$ are combined to produce an output 1430 in an output layer. The neural network performs simple processing via the hidden layer of simple processing elements, nodes $1420_1$, $1420_2$, $1420_3$, $1420_4$, which can exhibit complex global behavior, determined by the connections between the processing elements and element parameters.

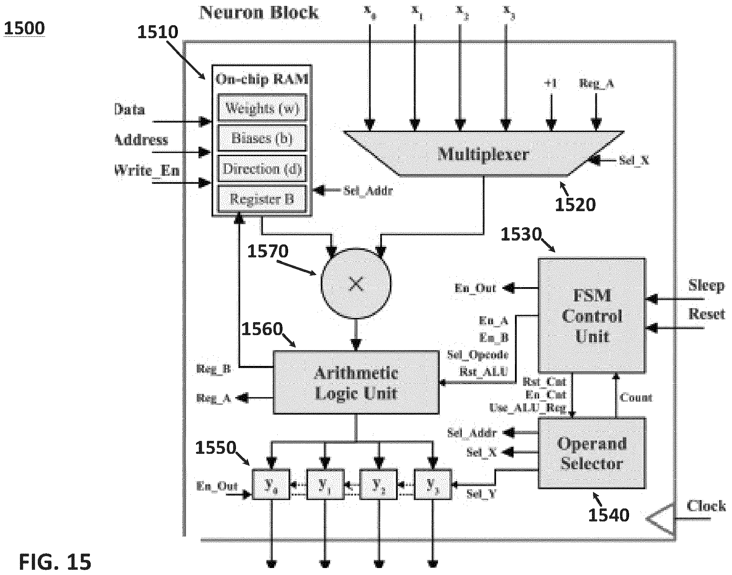

The neural network of FIG. 14 may be implemented in hardware. As depicted in FIG. 15 a hardware based neural network is depicted.

Cardiac arrhythmias, and atrial fibrillation in particular, persist as common and dangerous medical ailments, especially in the aging population. In patients with normal sinus rhythm, the heart, which is comprised of atrial, ventricular, and excitatory conduction tissue, is electrically excited to beat in a synchronous, patterned fashion. In patients with cardiac arrythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. Such abnormal conduction has been previously known to occur at various regions of the heart, for example, in the region of the sino-atrial (SA) node, along the conduction pathways of the atrioventricular (AV) node and the Bundle of His, or in the cardiac muscle tissue forming the walls of the ventricular and atrial cardiac chambers.

Cardiac arrhythmias, including atrial arrhythmias, may be of a multiwavelet reentrant type, characterized by multiple asynchronous loops of electrical impulses that are scattered about the atrial chamber and are often self-propagating. Alternatively, or in addition to the multiwavelet reentrant type, cardiac arrhythmias may also have a focal origin, such as when an isolated region of tissue in an atrium fires autonomously in a rapid, repetitive fashion.

One type of arrhythmia, atrial fibrillation, occurs when the normal electrical impulses generated by the sinoatrial node are overwhelmed by disorganized electrical impulses that originate in the atria and pulmonary veins causing irregular impulses to be conducted to the ventricles. An irregular heartbeat results and may last from minutes to weeks, or even years. Atrial fibrillation (AF) is often a chronic condition that leads to a small increase in the risk of death often due to strokes. Risk increases with age. Approximately 8% of people over 80 having some amount of AF. Atrial fibrillation is often asymptomatic and is not in itself generally life-threatening, but it may result in palpitations, weakness, fainting, chest pain and congestive heart failure. Stroke risk increases during AF because blood may pool and form clots in the poorly contracting atria and the left atrial appendage. The first line of treatment for AF is medication that either slow the heart rate or revert the heart rhythm back to normal. Additionally, persons with AF are often given anticoagulants to protect them from the risk of stroke. The use of such anticoagulants comes with its own risk of internal bleeding. In some patients, medication is not sufficient and AF is deemed to be drug-refractory, i.e., untreatable with standard pharmacological interventions. Synchronized electrical cardioversion may also be used to convert AF to a normal heart rhythm. Some AF patients are treated by catheter ablation.

A catheter ablation-based treatment may include mapping the electrical properties of heart tissue, especially the endocardium and the heart volume, and selectively ablating cardiac tissue by application of energy. Cardiac mapping, for example, creating a map of electrical potentials (a voltage map) of the wave propagation along the heart tissue or a map of arrival times (a local time activation (LAT) map) to various tissue located points, may be used for detecting local heart tissue dysfunction Ablations, such as those based on cardiac mapping, may cease or modify the propagation of unwanted electrical signals from one portion of the heart to another.

The ablation process damages the unwanted electrical pathways by formation of non-conducting lesions. Various energy delivery modalities have been disclosed for forming lesions, and include use of microwave, laser, irreversible electroporation via pulsed field ablation and more commonly, radiofrequency energies to create conduction blocks along the cardiac tissue wall. In a two-step procedure-mapping followed by ablation-electrical activity at points within the heart is typically sensed and measured by advancing a catheter containing one or more electrical sensors (or electrodes) into the heart, and acquiring data at a multiplicity of points. These data are then utilized to select the endocardial target areas at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems in order to reconstruct the anatomy of the heart chamber of interest.

For example, cardiologists rely upon software such as the Complex Fractionated Atrial Electrograms (CFAE) module of the CARTO®3 3D mapping system, produced by Biosense Webster, Inc. (Diamond Bar, Calif.), to analyze intracardiac EGM signals and determine the ablation points for treatment of a broad range of cardiac conditions, including atypical atrial flutter and ventricular tachycardia.

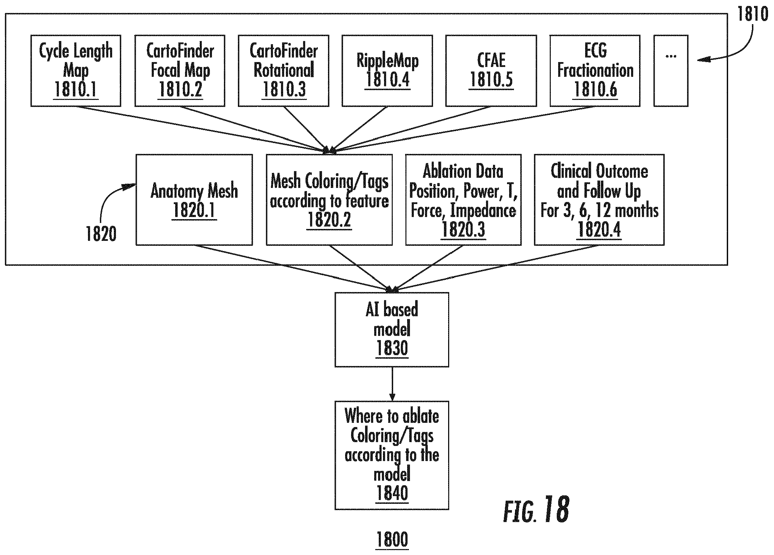

There are mapping algorithms that are generally used to map AFIB and there are mapping algorithm that are used to map VT. As for AFIB, the different mapping algorithms may include, but are not limited to, CFAE, Ripple frequency, Cycle length mapping, CARTOFINDER focal point, CARTOFINDER rotors, Low Voltage Zones. Other maps may include the cycle length map, Carto - Finder® focal map, Carto - Finder® rotational, Ripple Map4, CFAE, and ECG Fractionation as will be discussed in additional detail with respect to FIG. 18. The 3D maps can provide multiple pieces of information regarding the electrophysiological properties of the tissue that represent the anatomical and functional substrate of these challenging arrhythmias.

Electrode catheters have been in common use in medical practice for many years. They are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. RF (radio frequency) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees C., a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs and the catheter must be removed from the body and the tip electrode cleaned.

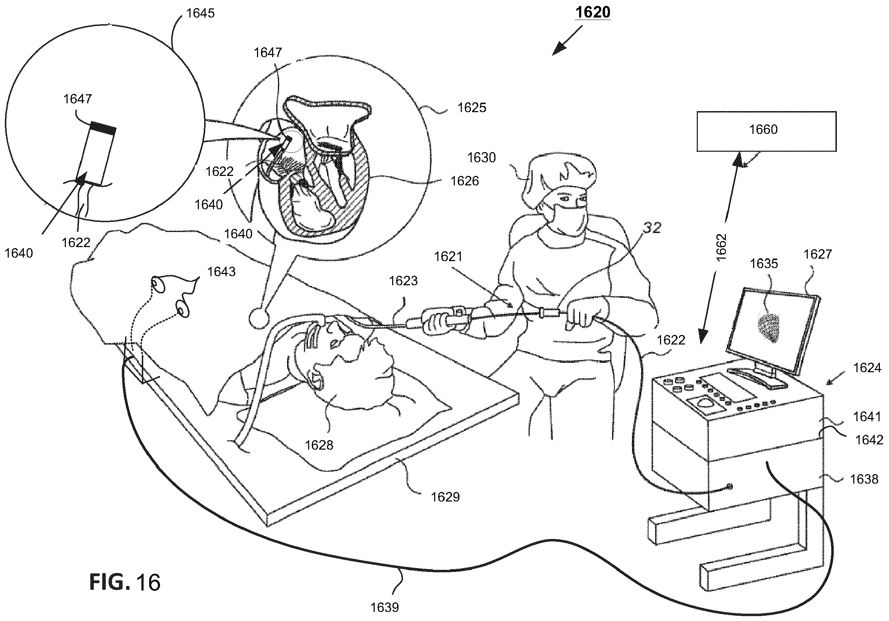

FIG. 16 is a diagram of an exemplary system 1620 in which one or more features of the disclosure subject matter can be implemented. All or parts of system 1620 may be used to collect information for a training dataset and/or all or parts of system 1620 may be used to implement a trained model. System 1620 may include components, such as a catheter 1640, that are configured to damage tissue areas of an intra-body organ. The catheter 1640 may also be further configured to obtain biometric data. Although catheter 1640 is shown to be a point catheter, it will be understood that a catheter of any shape that includes one or more elements (e.g., electrodes) may be used to implement the embodiments disclosed herein. System 1620 includes a probe 1621, having shafts that may be navigated by a physician 1630 into a body part, such as heart 1626, of a patient 1628 lying on a table 1629. According to embodiments, multiple probes may be provided, however, for purposes of conciseness, a single probe 1621 is described herein but it will be understood that probe 1621 may represent multiple probes. As shown in FIG. 16, physician 1630 may insert shaft 1622 through a sheath 1623, while manipulating the distal end of the shafts 1622 using a manipulator 1632 near the proximal end of the catheter 1640 and/or deflection from the sheath 1623. As shown in an inset 1625, catheter 1640 may be fitted at the distal end of shafts 1622. Catheter 1640 may be inserted through sheath 1623 in a collapsed state and may be then expanded within heart 1626. Cather 1640 may include at least one ablation electrode 1647 and a catheter needle 1648, as further disclosed herein.

According to exemplary embodiments, catheter 1640 may be configured to ablate tissue areas of a cardiac chamber of heart 1626. Inset 1645 shows catheter 1640 in an enlarged view, inside a cardiac chamber of heart 1626. As shown, catheter 1640 may include at least one ablation electrode 1647 coupled onto the body of the catheter. According to other exemplary embodiments, multiple elements may be connected via splines that form the shape of the catheter 1640. One or more other elements (not shown) may be provided and may be any elements configured to ablate or to obtain biometric data and may be electrodes, transducers, or one or more other elements.

According to embodiments disclosed herein, the ablation electrodes, such as electrode 1647, may be configured to provide energy to tissue areas of an intra-body organ such as heart 1626. The energy may be thermal energy and may cause damage to the tissue area starting from the surface of the tissue area and extending into the thickness of the tissue area.

According to exemplary embodiments disclosed herein, biometric data may include one or more of LATs, electrical activity, topology, bipolar mapping, dominant frequency, impedance, or the like. The local activation time may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 16, the probe 1621, and catheter 1640 may be connected to a console 1624. Console 1624 may include a processor 1641, such as a general-purpose computer, with suitable front end and interface circuits 1638 for transmitting and receiving signals to and from catheter, as well as for controlling the other components of system 1620. In some embodiments, processor 1641 may be further configured to receive biometric data, such as electrical activity, and determine if a given tissue area conducts electricity. According to an embodiment, the processor may be external to the console 1624 and may be located, for example, in the catheter, in an external device, in a mobile device, in a cloud-based device, or may be a standalone processor.

As noted above, processor 1641 may include a general-purpose computer, which may be programmed in software to carry out the functions described herein. The software may be downloaded to the general-purpose computer in electronic form, over a network, for example, or it may, alternatively or additionally, be provided and/or stored on non-transitory tangible media, such as magnetic, optical, or electronic memory. The example configuration shown in FIG. 16 may be modified to implement the embodiments disclosed herein. The disclosed embodiments may similarly be applied using other system components and settings. Additionally, system 1620 may include additional components, such as elements for sensing electrical activity, wired or wireless connectors, processing and display devices, or the like.

According to an embodiment, a display connected to a processor (e.g., processor 1641) may be located at a remote location such as a separate hospital or in separate healthcare provider networks. Additionally, the system 1620 may be part of a surgical system that is configured to obtain anatomical and electrical measurements of a patient's organ, such as a heart, and performing a cardiac ablation procedure. An example of such a surgical system is the Carto® system sold by Biosense Webster.

The system 1620 may also, and optionally, obtain biometric data such as anatomical measurements of the patient's heart using ultrasound, computed tomography (CT), magnetic resonance imaging (MRI) or other medical imaging techniques known in the art. The system 1620 may obtain electrical measurements using catheters, electrocardiograms (EKGs) or other sensors that measure electrical properties of the heart. The biometric data including anatomical and electrical measurements may then be stored in a memory 1642 of the mapping system 1620, as shown in FIG. 16. The biometric data may be transmitted to the processor 1641 from the memory 1642. Alternatively, or in addition, the biometric data may be transmitted to a server 1660, which may be local or remote, using a network 1662.

Network 1662 may be any network or system generally known in the art such as an intranet, a local area network (LAN), a wide area network (WAN), a metropolitan area network (MAN), a direct connection or series of connections, a cellular telephone network, or any other network or medium capable of facilitating communication between the mapping system 1620 and the server 1660. The network 1662 may be wired, wireless or a combination thereof. Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology generally known in the art. Additionally, several networks may work alone or in communication with each other to facilitate communication in the network 1662.

In some instances, the server 1662 may be implemented as a physical server. In other instances, server 1662 may be implemented as a virtual server a public cloud computing provider (e.g., Amazon Web Services (AWS) ®).

Control console 1624 may be connected, by a cable 1639, to body surface electrodes 1643, which may include adhesive skin patches that are affixed to the patient 1630. The processor, in conjunction with a current tracking module, may determine position coordinates of the catheter 1640 inside the body part (e.g., heart 1626) of a patient. The position coordinates may be based on impedances or electromagnetic fields measured between the body surface electrodes 1643 and the electrode 1648 or other electromagnetic components of the catheter 1640. Additionally, or alternatively, location pads may be located on the surface of bed 1629 and may be separate from the bed 1629.

Processor 1641 may include real-time noise reduction circuitry typically configured as a field programmable gate array (FPGA), followed by an analog-to-digital (A/D) ECG (electrocardiograph) or EMG (electromyogram) signal conversion integrated circuit. The processor 1641 may pass the signal from an A/D ECG or EMG circuit to another processor and/or can be programmed to perform one or more functions disclosed herein.

Control console 1624 may also include an input/output (I/O) communications interface that enables the control console to transfer signals from, and/or transfer signals to electrode 1647.

During a procedure, processor 1641 may facilitate the presentation of a body part rendering 1635 to physician 1630 on a display 1627, and store data representing the body part rendering 1635 in a memory 1642. Memory 1642 may comprise any suitable volatile and/or non-volatile memory, such as random-access memory or a hard disk drive. In some embodiments, medical professional 1630 may be able to manipulate a body part rendering 1635 using one or more input devices such as a touch pad, a mouse, a keyboard, a gesture recognition apparatus, or the like. For example, an input device may be used to change the position of catheter 1640 such that rendering 1635 is updated. In alternative embodiments, display 1627 may include a touchscreen that can be configured to accept inputs from medical professional 1630, in addition to presenting a body part rendering 1635.

Described herein relates to aiding a physician in locating an area on which to perform an ablation procedure in patients with AFIB.

When a physician commences an ablation procedure, there are certain areas or regions that are more optimal to perform the ablation than others. A model may be trained using inputs that output an optimal location for a physician to perform the ablation. For example, a mask is effectively generated, but it is not known which mask provides the best information to a physician to perform the ablation. Accordingly, the maps may be taken as an input for an actual ablation point to train the model for ML from the masks to generate an optimal ablation point.

The machine learning model may be trained by utilizing one of more of cardiac maps, EGM data, contact force data, respiration data, tissue proximity data, ablation location, ablation parameters and procedure outcome of retrospective procedures. Once trained, the machine learning model may predict the optimum ablation location and parameters given inputs, such as cardiac maps and EGM data. In some cases, the location may be important and in others the parameters may be important. That is, a physician may provide a location of the desired ablation and, given the cardiac maps and the desired ablation location, the system may infer optimal ablation parameters. In addition, the system may predict a success percentage at various ablation locations , (e.g., 60 percent, 70 percent etc.).

To train the system, the acute outcome of retrospective cases may be fed to the machine learning model. The outcome after a blanking period of several days may be used. The outcome may also be followed up for the long-term outcome and the long-term outcome of retrospective cases may be fed to the machine learning model to continue to train the model.

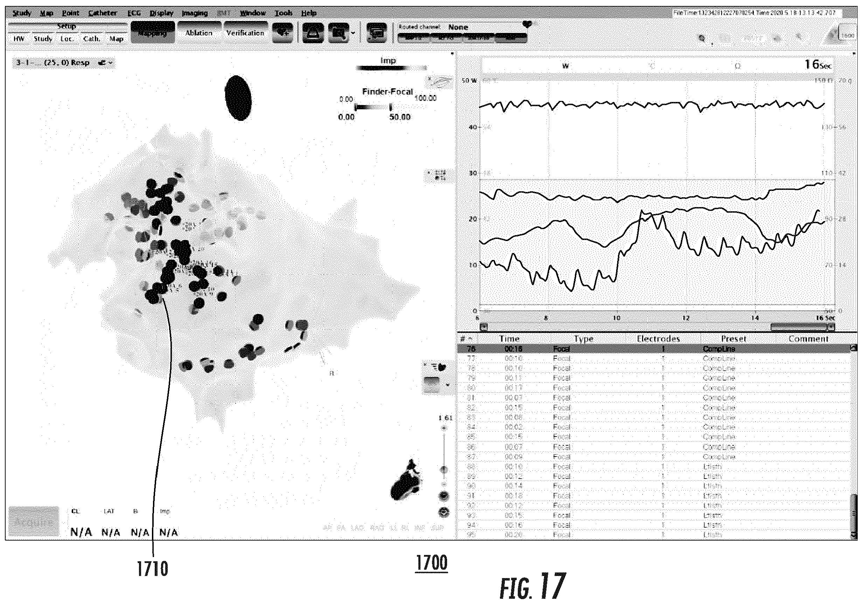

FIG. 17 is a diagram of a cardiac map 1700 of an exemplary retrospective study with locations 1710 where the physician performed ablations. As shown in FIG. 17, multiple ablation points 1710 may have been performed using different parameters and each utilizing various strategies. The information gained from data discussed below that is associated with these ablation points 1710 may be provided to the model to aid the model in learning and associating the data with each outcome allowing for prediction and suggestion of locations for a current or upcoming ablation, as well as an expected success at a particular location and parameters for use in the ablation.

FIG. 18 is a schematic diagram of an exemplary workflow 1800 for performing an ablation. The workflow 1800 includes a number of inputs 1810, such as the cycle length map 1810.1, Carto - Finder® focal map 1810.2, Carto - Finder® rotational 1810.3, Ripple Map 1810.4, CFAE 1810.5, and ECG Fractionation 1810.6. Other inputs 1810 may also be included as represented as input 1810.7 in FIG. 18. As can also be seen in FIG. 18, information 1820 may be provided to an AI based model 1830 (e.g., Anatomy Mesh 1820.1, Mesh Coloring/Tags 1820.2, Ablation Data 1820.3, Clinical Outcome and Follow-up 1820.4). Each information 1820 is a mapping that may include for each location point on the 3D mesh, the Value (real number), and may further include the start and end time points of the time period in which the catheter was located at that point. This timing information 1820 may therefore include the information that different points in the 3D mapping were calculated based on measurements done at different times. Further, if a certain point value was triangulated by the algorithm rather than obtained from a visit of the catheter at that point, then a triangulation of relevant time points may be used.

The input 1810 may include parameters about the patient, such as, age, gender, physical dimensions (of body and/or the heart and atria), Medical history including medicine usage, and the type of atrial fibrillation (one of several classes: Paroxysmal, Persistent or Long standing).

Model 1830 being provided inputs 1810 and information 1820, for example, may output an ablation location 1840 as well as parameters. The output including the ablation location 1840 is a 3D mapping of the mesh, where each point has a score between 0 and 1, indicating a recommendation strength of whether the point is a good candidate for ablation.

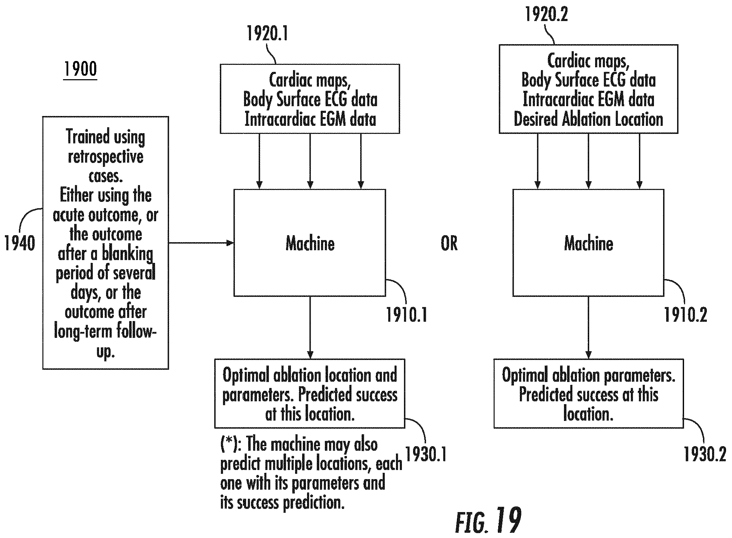

FIG. 19 is an example diagram of a schematic 1900 illustrating aiding a physician in locating an area to perform an ablation on patients with AFIB. The schematic 1900 uses machine learning (generally 1910) and includes the physician providing 1920.2 not providing 1920.1 an ablation location. The cardiac maps and EGM signals 1920.1, 1920.2 may be provided to the machine and the machine may be queried for the optimal ablation location and parameters 1930.1, 1930.2. The machine may also be provided a potential or desired ablation location 1920.2 and the machine may provide the ablation parameters 1930.2 for this provided location. The machine can also calculate the expected success of the ablation 1930.1, 1930.2.

The following provides further detail as to exemplary embodiments. A high-density basket catheter may be utilized to collect the intracardiac EGM from all the LA during a specific time (for example, during 1 minute). The system may be configured such that if the AFIB terminates immediately or a few days after the case, then the information may be provided to a neural network, other ablations the physician is involved in may also be provided to the neural network. Such a delay in reporting may capture AFIB situations including those where the AFIB does not terminate immediately, requiring a recovery period of multiple days for the arrythmia to disappear completely. In such procedures, it may be difficult to identify which ablation was at the critical site, however it is known that one of these ablation sites was the critical site. The neural network may learn to detect the critical ablation site. For example, assuming two patients with similar cardiac activity, the physician of the first patient made an ablation at locations A and B, the physician of the second patient made an ablation at locations B and C, the neural network can learn that this activity has a strong correlation with B (and not A and C).

The data clusters for recommending an ablation strategy can include, for example, Ripple Freq. maps (Ripple percentage and Peaks), Fragmentation index, Cycle length maps, CFAE, Finder, Fractionation map, Complexity map, Voltage map, Clinical Ablation parameters (site, index etc.), and Clinical outcome (acute and/or after a blanking period of several days and/or after long-term Follow-Up).

The model can be trained on studies and clinical outcomes in order to validate it on successful cases. Data for input in training the model for the machine includes the maps that are created during the ablation procedure of the specific physician, the ablation data collected during the procedure, ablation Catheter type, 3D Location of ablation points, power used for ablation, time of point ablation duration, irrigation, catheter stability, parameters related to the area of the ablation to verify transmural ablation based on the 'predicted' tissue width, images generated during the ablation based on the different CARTO Maps LAT, Voltage, Visitag, etc. taken at several fixed views, and the outcome of the procedure. Respiration, respiration prediction and indicators, ACL currents and TPI are inputs. Generally, the data may be obtained using intracardiac EGM signals, body surface ECG signals, applied force of the catheter, tissue thickness measured via CT or MRI, ripple percentage and peaks calculated in Ripple Maps, fragmentation/Fractionation/ECG Complexity index, cycle Length Maps, CFAE locations, electrical rotors and Focal activity calculated by Carto - Finder®, 3D location of ablation points, ablation time, power/temperature, the Tag Index values of the ablations, the radius and/or depth of the ablation, as predicted by an algorithm, the ablation catheter type, irrigation level during the ablation, catheter stability level during the ablation, parameters related to the area of the ablation to verify transmural ablation based on the 'predicted' tissue width, images generated during the ablation based on the different CARTO Maps LAT, Voltage, Visitag, etc. taken at several fixed views, characteristics of the pulsed field ablation, such as the voltage amplitude, pulse width, inter-phase delay, inter-pulse delay, pulse cycle length, and clinical outcome (acute, or after an approximately 2-days of a blanking period, or after a longer follow-up period, e.g. 12 months).

The data may be transferred in real-time (during the case) to a storage device in the cloud. The data may be transferred to a storage device in the cloud after the case, either automatically or when requested by the user. The system may transfer the data to a storage device in the cloud when the computer is idle. The user may manually transfer the data to a training center, where the model is trained, for example, all past cases stored in the workstation machine.

The AI model may be a supervised ML, use reinforcement learning and/or if images are analyzed, use a convolutional neural network. The model may be built in various ways. For example, if the case had a desired outcome, train the model with the ablation parameters as an output and all other parameters as an input. Give the ablation parameters as an output, and all other parameters as an input to the model, and perform reinforcement learning with the actual outcome. Give all the parameters to the model as inputs, the outcome as the output, and let the model learn to predict the outcome. All parameters and ablation location of cases with desired outcome may be used as inputs, ablation parameters can be an output.

Further a tag index, which is also called an ablation index may be calculated. An example formula is below, where K, a, b and c are constants, CF is the applied contact force of the ablation catheter, P is the constant power applied during the ablation and t is the duration of the ablation.

$$Index = \left( k * \int_0^t CF^a(\tau)P^b(\tau)d\tau \right)^c$$

CF: Contact Force, P: RF Power, t: Application Time

Again, the model may be trained by having the clinical outcome fed to the system. Either acute outcome may be used, and/or the outcome after a blanking period of several days, for example two days may be used, or the outcome after a longer follow-up period (for example, after 12 months) may be used. The system may suggest ablation location and parameters. Or, alternatively, the physician may provide the system a planned ablation location, and the system may suggest ablation parameters only, alongside with the expected success percent of this ablation. The physician may provide the system both the ablation location, and also the ablation parameters to be used, and the system may predict the expected outcome (ablation parameters may include the ablation power in Watts, the target temperature of the ablation in Celsius, ablation duration in seconds, or a single value summarizing all ablation parameters using some formula, such as the tag index formula described above).

The model learns which inputs are most important and which are less. The model may suggest an ablation location on the tissue, or an ablation strategy (such as, recommended temperature, power, ablation index/tag index, ablation duration). The locations may be marked on the computer screen over the map of the heart.

The training may be performed on one or more CPU, GPU or TPU processors, FPGA chips, or on ASIC dedicated to perform deep learning calculations. Also, it may be noted that the success of the ablation may be unknown until it is performed. For example, the catheter may move during an ablation and also some ablation parameters may be unknown until the ablation is done. The algorithm as suggested can integrate the prior information and the set of ablations accomplished and suggest the next ablation step as information becomes known.

The calculations may be performed, for example, inside the CARTO workstation machine, on a server in the hospital, on a server, and in the cloud, in an area owned by the hospital.

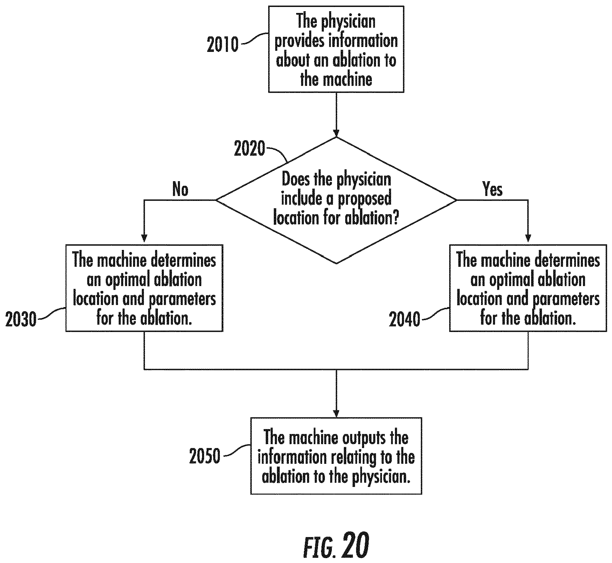

In accordance with the above, FIG. 20 is an example diagram of an exemplary method 2000 of aiding a physician in locating an area to perform an ablation on patients with AFIB using machine learning. In step 2010, the physician provides information about an ablation to the machine as described above. If the physician does not provide the machine with a location in step 2020, then the method proceeds to step 2030 where the machine utilizes the inputs from the physician described above and in FIG. 19, to analyze based on prior ablations and determine a location for the ablation as well as parameters to utilize during the performance of the ablation.

If the physician provides location information in step 2020, then the method proceeds to step 2040 where the machine utilizes the inputs from the physician described above and in FIG. 19, to analyze based on prior ablations and determine parameters to utilize during the performance of the ablation.

In step 2050, the machine provides the physician with the information related to the ablation to be performed. That is, if the physician did not provide a location, the machine provides both the location information and the parameters. If the physician provided an ablation location, the machine provides the parameters, without providing the location. In either case, the machine may also provide a preferred strategy to the physician based upon the inputs and the comparison to the strategies used previously in ablations in accordance with the data provided above.

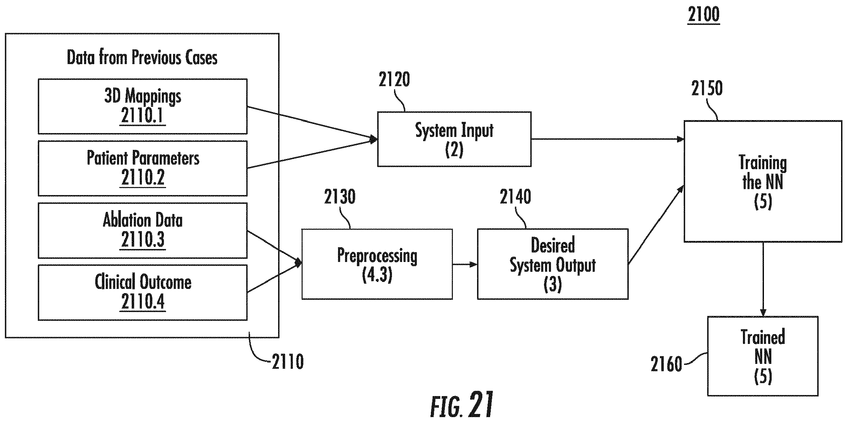

FIG. 21 illustrates the data preparation and training of the present system. Data from previous patient cases 2110 is used for training the system. for each such case. This data may include 3D mappings 2110.1 (described in detail herein above) and patient parameters 2110.2 data (described in detail herein below), such as age, gender, medical history, etc., to be used as input to the system described herein above and data to be used for calculation of the desired output. This data may include ablation data 2110.3 and clinical outcome 2110.4. Ablation data 2110.3 may include information such as the location of ablations that have been performed, and indications relating to procedure quality for each ablation point including stability, power, force, impedance, impedance change during the ablation, for example. The ablation data 2110.3 may include discounting ablation points that have a low procedural acceptance. Such discounting may include removing (discount of 100%), or minimizing the weight of such points, such as discounting by 10, 20, 25, 50, 66 % for example. Discounting % may be determined according to the ablation index that is defined in paragraph [0054] above, or according to a similar index that is based on one or several parameters relating to procedural quality.

Clinical outcome 2110.4 may include weighting previous data according to the survival duration of the procedure's results. For example, cases with AFIB recurrence of 3 months following the procedure may be assigned a lower weight than cases with AFIB which recurred after 12 months for the procedure, for example.

Preprocessing 2130 may occur to conform to the desired output form, namely: 3D mapping of the mesh, where each point has a score between 0 and 1, indicating a recommendation strength of whether the point is a good candidate for ablation. Calculating this score may rely on the "Ablation Index" as calculated by CARTO(R) for example, discounting ablation points that have low procedural quality (e.g. instability of the point, ablation which is not deep enough or does not use enough power), a particular patient's data set may be weighted according to the survival duration of the procedure's results. For example, cases with AFIB recurrence of 3 months following the procedure will have a lower weight than cases with AFIB which recurred only after 12 months following the procedure. After preprocessing 2130, the desired output 2140 may be included and provided to training the neural network at step 2150. Additionally, the inputs 2110 may be fed directly into training as a training set using system input 2120 and provided to training the neural network at step 2150. Once the training 2150 occurs, the neural network is trained 2160. The dataset may be split into a training set, a validation set, and a test set. The training and validation sets may be used during system development, while the test set is used for evaluating the system's accuracy. Also, cross-validation may be used to improve performance.

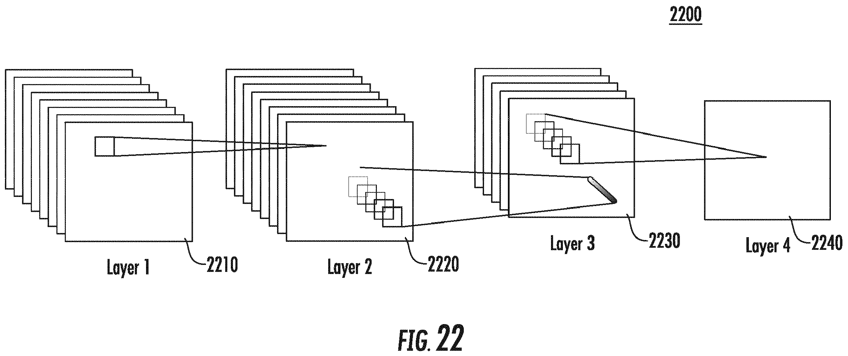

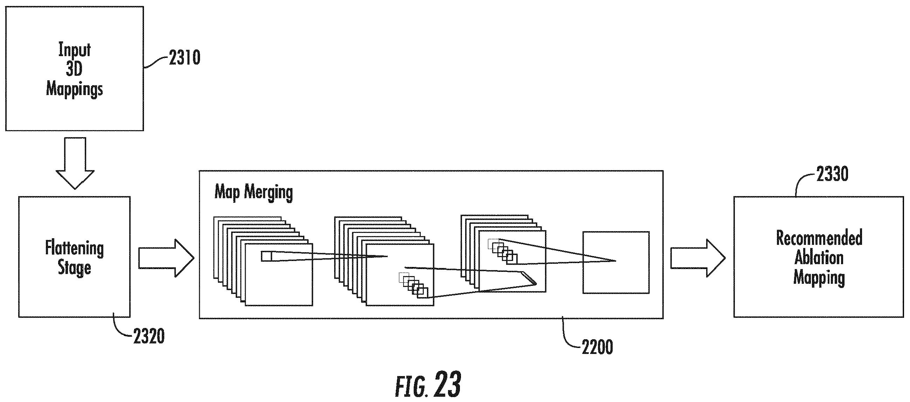

FIGs. 22 and 23 illustrate an example combined architecture for the present system. The mappings may be input into the system at step 2310 as described herein. Flattening may occur at step 2320. Flattening includes "flattening" 3D mapping of a cardiac mesh, i.e. translation of data points on the left atrium surface to a 2D mapping (in a similar way that the 3D surface of the Earth sphere can be projected to a 2D map), such as by stereographic projection or other types of projections, such as orthographic projection, Mercator projection, for example

The flattening provides the system a point on the mesh as a potential candidate for ablation, the decision depends on the input mapping values in the surrounding region of the candidate point, rather than the entire mapping. The calculation uses CNNs. CNNs as described above may be successfully trained and optimized to identify certain kinds of geometric "features" (e.g. a slanted line, a circle) based on the immediate surrounding region, regardless of the particular location where a feature appears in the 2D image.

There are many possible neural architectures as described above that may be used to calculate the desired output. More complex architectures may be desired, depending on the nature of the data.

By way of example, the NN architecture is the output of a pre-processing stage which flattens 2320 the 3D input mappings to 2D mappings. Thus, the input from flattening stage 2320 to map merging 2200 includes N mappings, each is a 2D "image" of size HxW providing one real value.

Map merging 2200 combines the input mappings by using a linear combination. For example, using one neural layer of size HxW, where neuron (i,j) is fed the values in position (i,j) in each of the N images. If each point in each input map provides, by itself, a somewhat reliable indication for good ablation, and if a simple linear combination / averaging of the input maps is sufficient for integration, then this configuration may be sufficient. The advantage is that it is easy to train, with less data compared to more complex models. However, the results may not be as accurate, and more complex models may be needed, as explained below.

Additional layers (deeper network) may be utilized allowing to represent more complex functions. Larger layers may also be utilized allowing to capture more nuances in the data. There may also be separate processing on each input map before combining information. Other kinds of layer architectures, e.g. fully-connected, CNN, max-pooling, etc. may also be utilized.

Returning to the input to map merging 2200 from flattening stage 2320, in an example network, the input includes N=8 2D mappings, for example (each obtained by flattening the output of a different algorithm 2320, such as CFAE, Cycle Length Map, etc.). Each such mapping is processed separately in Layer 1 2210 by a different CNN grid. Each of the input N images may be processed using individualized convolution models, namely each input image may be provided into a separate CNN. The output of each CNN is an image that gives a preliminary output recommendation map based on just one input map. Further layers may be applied on each map "track" separately, according to standard art in Deep Learning for image processing, i.e. CNN, max-pooling, and other paradigms.

In the example, the output of layer 1 2210 may include N=8 convoluted mappings, which are fed into Layer 2 2220. These mappings may be provided to the combining Layer 3 2230, which has depth k=5 in order to allow for non-linear combinations. The combining layer 2230 receives the input maps and/or the output of the previous separate-processing layers and combines them into a single representation. The combination may be done using a simple linear combination, or using more complex combinations, namely non-linear combination, and/or CNN, max-pooling, and other standard layers of image processing.

For example, the system may be enhanced by using two layers: a first layer of Layer 3 (not shown) of size HxWxk (i.e. a 2D layer HxW with "thickness" k > 1), and a second layer of Layer 3 (not shown) of size HxW. Each of the k neurons in position (i,j) in the first layer is fed the N signals from position (i,j) from each of the N input maps, and its output is given to neuron (i,j) in the second layer. Thus, non-linear combinations of the N values in each point may be represented.

Finally, the k combined layers are merged into the output map by Layer 4 2240. The values N=8 and k=5 here are merely example values. Also, further (or possibly fewer) layers than shown may be used, depending on the nature of the data and the required accuracy of the output.

Patient parameters, such as age, gender, medications, medical history and type of atrial fibrillation may affect the results. A separate model may be trained based on some of the input patient parameters. Alternatively, the patient inputs may be provided to the layers in the NN architecture to help it learn differences based on these parameters.

Hearts of different patients may be different, resulting in variations in the recorded data. According to an embodiment, the system may need to be trained in batches with each of the batches limited to the data of a single patient. Data needs to be collected from at least a certain number of patients for the system's training to be robust.

Even after the system is ready and is deployed in hospitals, additional data may be accumulated. It should be added to the training dataset, and the system should be re-trained, to continually improve its accuracy. Specifically, data from additional operations can provide feedback, by considering success ratings of ablations performed in accordance with the system's recommendations.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. Examples of computer-readable media include electronic signals (transmitted over wired or wireless connections) and computer-readable storage media. Examples of computer-readable storage media include, but are not limited to, a read only memory (ROM), a random-access memory (RAM), a register, cache memory, semiconductor memory devices, magnetic media such as internal hard disks and removable disks, magneto-optical media, and optical media such as CD-ROM disks, and digital versatile disks (DVDs). A processor in association with software may be used to implement a radio frequency transceiver for use in a WTRU, UE, terminal, base station, RNC, or any host computer.

**Claims**

1. A method of aiding a physician in locating an area to perform an ablation on patients with atrial fibrillation (AFIB), comprising:

   receiving data at a machine, from at least one device, the data including information relating to a desired location for performing an ablation;
   generating, by the machine, an optimal location for performing the ablation based upon the data and inputs; and
   providing an optimal location for performing the ablation output by the model.

2. The method of claim 1 wherein the receiving includes cardiac maps, and the providing includes the optimal ablation location and parameters.

3. The method of claim 1 wherein the receiving includes cardiac maps and EGM signals, and the providing includes the optimal ablation location and parameters.

4. The method of claim 1 wherein the receiving includes cardiac maps and EGM signals and a preferred ablation location, and the providing includes the parameters for ablating at the preferred ablation location.

5. The method of claim 1 wherein the receiving includes cardiac maps and EGM signals and a preferred ablation location and the ablation parameters, and the providing includes the parameters for ablating at the preferred ablation location.

6. A system for aiding a physician in locating an area to perform an ablation on patients with atrial fibrillation (AFIB), the system comprising:

   a first stage receiving data from at least one device, the data including information relating to a desired location for performing an ablation;
   a second stage generating an optimal location for performing the ablation based upon the data and inputs; and
   the system outputting an optimal location for performing the ablation.

7. The system of claim 6 wherein the first stage receives cardiac maps, and the system outputs the optimal ablation location and parameters.

8. The system of claim 6 wherein the first stage receives cardiac maps and EGM signals, and the system outputs the optimal ablation location and parameters.

9. The system of claim 6 wherein the first stage receives cardiac maps and EGM signals and a preferred ablation location, and the system outputs the parameters for ablating at the preferred ablation location.

10. The system of claim 6 wherein the first stage receives cardiac maps and EGM signals and a preferred ablation location and the ablation parameters, and the system outputs the parameters for ablating at the preferred ablation location.

11. The method of claim 1 or the system of claim 6 wherein the system is trained with acute outcomes of retrospective cases fed to the machine learning model.

12. The method of claim 1 or the system of claim 6 wherein training data includes any of the following: the maps that are created during the ablation procedure of the specific physician, the ablation data collected during the procedure, Ablation Catheter type, 3D Location of ablation points, Power used for ablation, Time of point ablation duration, Irrigation, Catheter stability, Parameters related to the area of the ablation to verify transmural ablation based on the 'predicted' tissue width, and/or the outcome of the procedure.

13. The method of claim 1 or the system of claim 6 wherein data clusters for recommending an ablation strategy include any of the following: Ripple Freq. maps with Ripple percentage and Peaks, Fragmentation index, Cycle length maps, CFAE, Finder, Fractionation map, Complexity map, Clinical Ablation parameters including site, and index, Clinical outcome including acute, after a blanking period of several days and after long-term Follow-Up.

14. The method of claim 1 or the system of claim 6 wherein data clusters for recommending an ablation strategy include patient parameters including at least one of age, gender, medications, medical history.

15. The method of claim 1 or the system of claim 6 wherein data clusters for recommending an ablation strategy include cardiac mapping including at least one of Ripple Freq. maps with Ripple percentage and Peaks, Fragmentation index, Cycle length maps, CFAE, Finder, Fractionation map, Complexity map, anatomical mapping including CT, MRI, ultrasonography of the heart, clinical ablation parameters including site, and index, and clinical outcome including acute, after a blanking period of several days and after long-term follow-up.

FIG. 1

EP 3 937 181 A1

FIG. 2

EP 3 937 181 A1

EP 3 937 181 A1

100

FIG. 3

FIG. 4

400

410 Data

420

430 Building a Model

440 Predicting Outcome

450 Training the Machine

Hardware

<u>500</u>

```
┌─────────────────────────────────┐
│   Collect Data From Hardware    │──∼510
└─────────────────────────────────┘
                 │
                 ▼
┌─────────────────────────────────┐
│   Train a Machine on the Hardware │──∼520
└─────────────────────────────────┘
                 │
                 ▼
┌─────────────────────────────────┐
│          Build a Model          │──∼530
└─────────────────────────────────┘
                 │
                 ▼
┌─────────────────────────────────┐
│  Predict Outcomes of the Hardware │──∼540
└─────────────────────────────────┘
```

# FIG. 5

EP 3 937 181 A1

610

| Weather | Play |
|---------|------|
| Sunny | No |
| Overcast | Yes |
| Rainy | Yes |
| Sunny | Yes |
| Sunny | Yes |
| Overcast | Yes |
| Rainy | No |
| Rainy | No |
| Sunny | Yes |
| Rainy | Yes |
| Sunny | No |
| Overcast | Yes |
| Overcast | Yes |
| Rainy | No |

620

| Frequency Table | | |
|-----------------|------|------|
| Weather | No | Yes |
| Overcast | | 4 |
| Rainy | 3 | 2 |
| Sunny | 2 | 3 |
| Grand Total | 5 | 9 |

630

| Likelihood Table | | | | |
|------------------|------|------|--------|------|
| Weather | No | Yes | | |
| Overcast | | 4 | =4/14 | 0.29 |
| Rainy | 3 | 2 | =5/14 | 0.36 |
| Sunny | 2 | 3 | =5/14 | 0.36 |
| All | 5 | 9 | | |
| | =5/14 | =9/14 | | |
| | 0.36 | 0.64 | | |

FIG. 6

Weather 710

Sunny 712 — Overcast 714 — Rainy 716

Overcast → Yes 715

Temperature 720

High 722 → No 723

Normal 724 → Yes 725

Temperature 730

Normal 732 → No 733

Low 734 → Yes 735

FIG. 7

FIG. 8

EP 3 937 181 A1

FIG. 9

FIG. 10

FIG. 11

FIG. 12

Bagging - Parallel                    Boosting - Sequential

FIG. 13

EP 3 937 181 A1

FIG. 14

FIG. 15

FIG. 16

1710     1700

FIG. 17

EP 3 937 181 A1

FIG. 18

1800

1900

1940 — Trained using retrospective cases. Either using the acute outcome, or the outcome after a blanking period of several days, or the outcome after long-term follow-up.

1920.1 — Cardiac maps, Body Surface ECG data Intracardiac EGM data

Machine — 1910.1

Optimal ablation location and parameters. Predicted success at this location.

(*): The machine may also predict multiple locations, each one with its parameters and its success prediction. — 1930.1

OR

1920.2 — Cardiac maps, Body Surface ECG data Intracardiac EGM data Desired Ablation Location

Machine — 1910.2

Optimal ablation parameters. Predicted success at this location. — 1930.2

FIG. 19

2010 — The physician provides information about an ablation to the machine

2020 — Does the physician include a proposed location for ablation?

No

2030 — The machine determines an optimal ablation location and parameters for the ablation.

Yes

2040 — The machine determines an optimal ablation location and parameters for the ablation.

2050 — The machine outputs the information relating to the ablation to the physician.

FIG. 20

FIG. 21

2200

Layer 4 — 2240

Layer 3 — 2230

Layer 2 — 2220

Layer 1 — 2210

*FIG. 22*

FIG. 23

Europäisches Patentamt
European Patent Office
Office européen des brevets

# EUROPEAN SEARCH REPORT

Application Number

EP 21 18 3847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/212833 A1 (UNIV LELAND STANFORD JUNIOR [US]) 7 November 2019 (2019-11-07) * paragraphs [0164] - [0181] * | 1-15 | INV. G16H20/40 |
| X | WO 2019/210092 A1 (VEKTOR MEDICAL INC [US]) 31 October 2019 (2019-10-31) * paragraphs [0071] - [0080] * | 1-15 | |
| X | US 2015/294082 A1 (PASSERINI TIZIANO [US] ET AL) 15 October 2015 (2015-10-15) * paragraphs [0025] - [0053] * | 1-15 | |
| X | Alhusseini Mahmood ET AL: "Arrhythmias and Clinical EP MACHINE LEARNING IDENTIFIES SITES WHERE ABLATION TERMINATES PERSISTENT ATRIAL FIBRILLATION", , 16 March 2019 (2019-03-16), page 301, XP055862038, Retrieved from the Internet: URL:https://www.jacc.org/doi/abs/10.1016/S 0735-1097%2819%2930909-X [retrieved on 2021-11-16] * the whole document * | 1-15 | |

TECHNICAL FIELDS SEARCHED (IPC)

G16H
A61B

The present search report has been drawn up for all claims

| Place of search | Date of completion of the search | Examiner |
|---|---|---|
| Berlin | 17 November 2021 | Trachterna, Morten |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 18 3847

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2021

| Patent document cited in search report | | | Publication date | Patent family member(s) | | | Publication date |
|---|---|---|---|---|---|---|---|
| WO 2019212833 | A1 | | 07-11-2019 | AU | 2019262858 | A1 | 10-12-2020 |
| | | | | CA | 3098979 | A1 | 07-11-2019 |
| | | | | CN | 112639990 | A | 09-04-2021 |
| | | | | EP | 3788588 | A1 | 10-03-2021 |
| | | | | JP | 2021521964 | A | 30-08-2021 |
| | | | | US | 2021236053 | A1 | 05-08-2021 |
| | | | | WO | 2019212833 | A1 | 07-11-2019 |
| WO 2019210092 | A1 | | 31-10-2019 | AU | 2019257716 | A1 | 26-11-2020 |
| | | | | CA | 3080944 | A1 | 31-10-2019 |
| | | | | CN | 112437964 | A | 02-03-2021 |
| | | | | CN | 112768077 | A | 07-05-2021 |
| | | | | EP | 3619720 | A1 | 11-03-2020 |
| | | | | EP | 3654349 | A1 | 20-05-2020 |
| | | | | JP | 2021522052 | A | 30-08-2021 |
| | | | | KR | 20210016359 | A | 15-02-2021 |
| | | | | WO | 2019210090 | A1 | 31-10-2019 |
| | | | | WO | 2019210092 | A1 | 31-10-2019 |
| US 2015294082 | A1 | | 15-10-2015 | NONE | | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63048830